# EUROPEAN PATENT SPECIFICATION

(11) **EP 4 392 085 B1**
(45) Date of publication and mention of the grant of the patent: **11.11.2026**
(21) Application number: 22862438.3
(22) Date of filing: 02.09.2022
(51) Int. Cl.: A61L 27/20, A61L 27/52, A61L 27/58, C08J 3/09, A61K 31/717, A61K 47/02, A61K 47/18, A61K 8/04, A61K 8/23, A61K 8/42, A61K 8/73, A61K 9/00, A61K 9/06, A61P 19/02, A61Q 19/08, C08B 15/00

(54) **INJECTABLE COMPOSITION**
INJIZIERBARE ZUSAMMENSETZUNG
COMPOSITION INJECTABLE

(30) Priority: 02.09.2021 AU 2021902847
(43) Date of publication of application: 03.07.2024
(73) Proprietor: The University of Queensland, St Lucia, Queensland 4072 (AU); Bulugudu Limited, Mount Isa, QLD 4825 (AU)
(72) Inventor: MARTIN, Darren, James, St Lucia, Queensland 4072 (AU); GRIFFITHS, Ian, St Lucia, Queensland 4072 (AU)
(74) Representative: Barker Brettell LLP
(86) International application number: PCT/AU2022/051077
(87) International publication number: WO 2023/028664

(56) References cited:
- AU-A4- 2020 100 319
- US-A1- 2014 010 790
- US-A1- 2014 010 790
- HOSSEINMARDI ALIREZA ET AL: "Reinforcement of natural rubber latex using lignocellulosic nanofibers isolated from spinifex grass", vol. 9, no. 27, 1 January 2017 (2017-01-01), United Kingdom, pages 9510 - 9519, XP093043226, ISSN: 2040-3364, Retrieved from the Internet <URL:https://pubs.rsc.org/en/content/articlepdf/2017/nr/c7nr02632c> DOI: 10.1039/C7NR02632C
- HOSSEINMARDI ALIREZA, ANNAMALAI PRATHEEP K., WANG LIANZHOU, MARTIN DARREN, AMIRALIAN NASIM: "Reinforcement of natural rubber latex using lignocellulosic nanofibers isolated from spinifex grass", NANOSCALE, ROYAL SOCIETY OF CHEMISTRY, UNITED KINGDOM, vol. 9, no. 27, 1 January 2017 (2017-01-01), United Kingdom , pages 9510 - 9519, XP093043226, ISSN: 2040-3364, DOI: 10.1039/C7NR02632C

## Description

### TECHNICAL FIELD

The present invention relates, *inter alia,* to injectable compositions formed from or comprising cellulosic fibers for cosmetic use or for use in plasma rich platelet therapy, and to cosmetic uses of the compositions.

### BACKGROUND ART

It will be clearly understood that, if a prior art publication is referred to herein, this reference does not constitute an admission that the publication forms part of the common general knowledge in the art in Australia or in any other country. While the following discussion particularly concerns dermal fillers, the subject matter of the application is not limited to this use.

Dermal fillers (also known as soft tissue fillers) are used to add fullness or smoothness to areas of the skin, especially in the face. This may be particularly desired where there has been volume loss, especially due to aging. Many visible signs of aging can be attributed to volume loss. Thinning of the skin due to aging is common, for example, in the cheeks, lips and around the mouth. Consequently, dermal filler is frequently used to replace lost volume in areas including the lips, nasolabial folds, perioral rhytids and cheeks.

Dermal filler is typically injected directly into the area to be treated, often after application of a topical anaesthetic cream. The effect of the dermal filler is immediate, and may last between 2 and 18 months, depending on the type of product, the treated area, and individual patient factors such as metabolism and if the patient has had previous treatments.

One of the most common types of dermal fillers is hyaluronic acid, which is estimated to comprise up to 80% of the dermal filler market. Hyaluronic acid has a good safety profile as it is found naturally in the body, and results are temporary as after a period of time the body gradually and naturally absorbs the hyaluronic acid. To achieve the desired consistency and longevity, hyaluronic acid products are synthetically cross-linked, and the degree of cross-linking can result in a variety of dermal filler products based on anatomical requirements. However, such cross-linked products can result in thicker gels, which can result in more challenging injectability, greater patient discomfort, and at certain thresholds, limitations on their use. Furthermore, such cross-linked products may form discrete particles, and even if such particles are hundreds of microns in diameter, they may still be felt in tissue. Consequently, there is a need for the development of alternative dermal fillers.

Hyaluronic acid is also found naturally in tissues throughout the body. In particular, hyaluronic acid performs a joint lubricating function, and therapeutically hyaluronic acid has been used to improve joint function, for example due to arthritis. In particular, hyaluronic acid has been used to treat osteoarthritis of the knee via intra-articular injection.

### SUMMARY OF INVENTION

The scope of protection is defined by the claims.

With the foregoing in view, the present invention in one embodiment is directed towards an injectable composition that may be useful as a dermal filler. In a further embodiment, the present invention which may at least partially overcome at least one of the abovementioned disadvantages or provide the consumer with a useful or commercial choice.

In a first aspect (not according to the claimed invention), there is disclosed an injectable composition formed from cellulosic fibers and a deep eutectic solvent. In one embodiment, the composition is a homogenized composition. In one embodiment, the composition is a mechanically refined composition, such as a milled or ultrasonicated composition. In another embodiment, the injectable composition is formed from cellulosic nanofibers and a deep eutectic solvent.

In a second aspect (not according to the claimed invention), there is disclosed an injectable composition comprising deep eutectic solvent treated cellulosic fibers. In one embodiment, the deep eutectic solvent treated cellulosic fibers are deep eutectic solvent treated cellulosic nanofibers.

In a third aspect (not according to the claimed invention), there is disclosed an injectable composition, the composition comprising cellulosic nanofibers. The cellulosic nanofibers may be treated or complexed with a deep eutectic solvent (DES). In one embodiment, the cellulosic nanofibers may be DES treated cellulosic nanofibers.

Features of the first to third aspects may be as described below.

As used herein, the terms "deep eutectic solvent treated cellulosic fibers" or "deep eutectic solvent treated cellulosic nanofibers" or "DES treated cellulosic nanofibers" or the like does not mean that the composition comprises a deep eutectic solvent. Rather, the term means that cellulosic fibers or cellulosic nanofibers has been treated or complexed with a deep eutectic solvent.

The term "deep eutectic solvent" would be known to a skilled person. For example, a review of deep eutectic solvents is provided in Smith, E.L. et al. (2014) Chemical Reviews, 114, 11060-11082. Deep Eutectic Solvents (or DESs) are systems formed from a eutectic mixture of Lewis or Brønsted acids and bases. They can contain a variety of anionic and/or cationic species. The DES may be a type I, a type II, a type III or a type IV DES.

The DES may be formed from a Lewis acid and a Lewis base. The Lewis base may include a nitrogen atom, or an amide group, or a urea group, or a carbamate group, or an ammonium group (such as a quaternary ammonium salt). The Lewis base may be urea or acetamide, especially urea. The Lewis acid may comprise an ammonium, phosphonium or sulfonium cation, an amine, an amide, a carboxylic acid, or a polyol; especially an ammonium cation. The Lewis acid may be sulfamic acid. In one embodiment, the Lewis acid is sulfamic acid, and the Lewis base is urea. In one embodiment, the Lewis acid is choline chloride. In one embodiment, the Lewis acid is choline chloride and the Lewis base is urea.

The Lewis acid and Lewis base may be present in the DES in any suitable molar ratio. In one embodiment, the molar ratio of Lewis acid : Lewis base is from 1:5 to 1:1, or from 1:4 to 1:2 or about 1:3. The molar ratio of Lewis acid : Lewis base may be 1:5, 1:4, 1:3, 1:2, or 1:1, especially 1:4, 1:3 or 1:2.

The deep eutectic solvent may modify the cellulosic fibers (or cellulosic nanofibers). For example, the deep eutectic solvent may sulfate the cellulosic fibers. Without wishing to be bound by theory, the deep eutectic solvent may modify the cellulosic fibers or nanofibers by ionic or covalent bonding, complexation or by dissolution of components of the cellulosic fibers that are then removed with the deep eutectic solvent.

In one embodiment, the composition comprises substantially no deep eutectic solvent; especially no deep eutectic solvent. The composition may be substantially free of residual deep eutectic solvent and its constituent components. The terms "substantially no" or "substantially free" means that the amount of deep eutectic solvent (or its constituent components) in the composition is below levels of toxicity for the deep eutectic solvent (or its constituent components). For example, the composition may comprise less than 2% by weight deep eutectic solvent (or its constituent components); especially less than 1.5%, 1%, 0.9%, 0.8%, 0.7%, 0.6%, 0.5%, 0.4%, 0.3%, 0.2%, 0.1% or 0.05% by weight deep eutectic solvent (or its constituent components).

When the composition is formed (or the cellulosic fibers or nanofibers are treated) using a deep eutectic solvent comprising sulfamic acid, the amount of sulfamic acid in the composition is less than 150 mg/L, 50 mg/L, 25 mg/L, 10 mg/L, 5 mg/L, 1 mg/L, 0.5 mg/L, 0.3 mg/L, 0.1 mg/L, 0.08 mg/L, 0.05 mg/L, 0.03 mg/L or 0.01 mg/L.

When the composition is formed (or the cellulosic fibers or nanofibers are treated) using a deep eutectic solvent comprising urea, the amount of urea in the composition is less than 20 mg/L, 10 mg/L, 5 mg/L, 2.5 mg/L, 1 mg/L, 0.8 mg/L, 0.5 mg/L, 0.3 mg/L, 0.1 mg/L, 0.08 mg/L, 0.05 mg/L, 0.03 mg/L or 0.01 mg/L.

Advantageously the inventors have found that the compositions of the first to third aspects have favourable rheological properties, mechanical integrity and from experiments that have been conducted appear to be safe for use and biocompatible, being broken down or absorbed by the body gradually over time. The cellulosic fibers (or nanofibers) also may be derived from a secure and abundant plant source. The compositions may also be injectable through a 30 or 31 gauge needle (as is current practice for hyaluronic acid compositions). Lastly, if an injection of the composition is erroneous, then an enzyme may be used to rapidly break down the cellulosic nanofibers *in situ.*

The composition is an injectable composition for medical or cosmetic use. The composition may be suitable for injection into a person or animal for the purpose of a medical procedure, for example to treat, prevent or ameliorate a condition, disorder or disease. The composition may be suitable for injection into a person or animal for the purpose of a cosmetic procedure, for example, as a dermal filler. In one embodiment, the composition comprises a therapeutic agent. In another embodiment, the composition does not comprise a therapeutic agent. In one embodiment, the composition is pharmacologically inert.

The cellulosic fibers (or nanofibers) may be bleached cellulosic fibers (or nanofibers). Suitable bleaching agents would be known to a skilled person. In one embodiment, the bleaching agent may be a chlorite or hypochlorite, especially a chlorite. The bleaching agent may be in an acidic solution, such as an acidic solution of chlorite. The bleaching agent may be sodium chlorite. The cellulosic fibers (or nanofibers) may be delignified. The cellulosic fibers (or nanofibers) may be bleached, delignified cellulosic fibers (or nanofibers). In some embodiments, the cellulosic fibers (or nanofibers) are bleached and/or delignified fibers of one or more of the plants described in paragraphs [0024-0027].

The cellulosic fibers (or cellulosic nanofibers) may be derived from a plant of the subtribe *Triodiinae.* The plant of the subtribe *Triodiinae* may be a plant of the genera *Triodia, Monodia* or *Symplectrodia;* especially a plant of the genera *Triodia.* The cellulosic fibers (or cellulosic nanofibers) may be derived from a drought-tolerant grass species, or an arid grass species.

The cellulosic fibers (or cellulosic nanofibers) may be derived from an Australian spinifex grass. Spinifex (also known as 'porcupine' and 'hummock' grass) is the long-established common name for three genera which includes *Triodia, Monodia* and *Symplectrodia* (not to be confused with the grass genus *Spinifex* that is restricted to coastal dune systems in Australia). Hummock grassland communities in arid Australia are dominated by spinifex species of the genus *'Triodia'.* There around 70 described species of *Triodia,* which are long-lived and deep rooted allowing root growth to penetrate through tens of metres under the ground. Of the species, abundant species are two soft species called *T. pungens, T. shinzii* and two hard species *T. basedowii, T. longiceps. T. Pungens* has been found to have a composition of approximately: cellulose (37 %), hemicellulose (36 %), lignin (25%) and ash (4%) in the un-washed form, such that hemicellulose content makes up 37% of the lignocellulosic content.

In one embodiment, the cellulosic fibers (or cellulosic nanofibers) is derived from a grass species having C4 leaf anatomy. Exemplary grasses having a C4 leaf anatomy includes the Australian spinifex grass discussed in the preceding paragraph. Examples of other grasses with C4 leaf anatomy that may be used to form the cellulosic nanofibers include *Digitaria sanguinalis (L.) Scopoli, Panicum coloratum L. var. makarikariense Goossens, Brachiaria brizantha (Hochst. Ex A. Rich) Stapf, D. violascens Link, P. dichotomiflorum Michaux, B. decumbens Stapf, Echinochloa crus-galli P. Beauv., P. miliaceum L., B. humidicola (Rendle) Schweick., Paspalum distichum L., B. mutica (Forsk.) Stapf, Setaria glauca (L.) P. Beauv, Cynodon dactylon (L.) Persoon, Panicum maximum Jacq., S. viridis (L.) P. Beauv, Eleusine coracana (L.) Gaertner, Urochloa texana (Buckley) Webster, Sorghum sudanense Stapf, E. indica (L.) Gaertner, Spodiopogon cotulifer (Thunb.) Hackel, Eragrostis cilianensis(Allioni) Vignolo-Lutati, Chloris gayana Kunth, Eragrostis curvula, Leptochloa dubia, Muhlenbergia wrightii, E. ferruginea (Thunb.) P. Beauv., Sporobolus indicus R. Br. Var.purpureo-suffusus (Ohwi) T. Koyama, Andropogon gerardii, Leptochloa chinensis (L.) Nees,* grasses of the *Miscanthus* genus (elephant grass), plants of the genus *Salsola* including Russian Thistle, ricestraw, wheat straw, corn stover, and *Zoysia tenuifolia Willd.*

Since the *Triodia* grasses are grown under arid conditions, the present inventors believe that other arid grasses that grow in Australia and other parts of the world may also be used in the present invention. The most drought tolerant grass genera, in Australia, (though they need water in their first 1 or 2 years) include Anigozanthos, Austrodanthonia, Austrostipa, Baloskion pallens, Baumea juncea, Bolboschoenus, Capillipedium, Carex bichenoviana, Carec gaudichaudiana, Carex appressa, C. tereticaulis, Caustis, Centrolepis, Chloris truncate, Chorizandra, Conostylis, Cymbopogon, Cyperus, Desmocladus flexuosa, Dichanthium sericeum, Dichelachne, Eragrostis, Eurychorda complanata, Evandra aristata, Ficinia nodosa, Gahnia, Gymnoschoenus sphaerocephalus, Hemarthria uncinata, Hypolaeana, Imperata cylindrical, Johnsonia, Joycea pallid, Juncus, Kingia australis, Lepidosperma, Lepironia articulate, Leptocarpus, Lomandra, Meeboldina, Mesomelaena, Neurachne alopecuroidea, Notodanthonia, Patersonia, Poa, Spinifex, Themedo triandra, Tremulina tremula, Triglochin, Triodia and Zanthorrhoea. Arid grasses that grow in other parts of the world that may also be using the present invention include *Aristida pallens* (Wire grass), *Andropogon gerardii* (Big bluestem), *Bouteloua eriopoda* (Black grama), *Chloris roxburghiana* (Horsetail grass), *Themeda triandra* (Red grass), *Panicum virgatum* (Switch grass), *Pennisetum ciliaris* (Buffel grass), *Schizachyrium scoparium* (Little bluestem), *Sorghatrum nutans* (Indian grass) and *Stipa tenacissima* (Needle grass).

Along with cellulose and lignin, hemicellulose is believed to be a key component of the various materials that constitute the cellulosic fibers (or cellulosic nanofibers). Without wishing to be bound by theory, it is believed that hemicellulose is likely distributed throughout the cellulosic fibers (or nanofibers) both on the surface of the fibers and in between the primary (elementary) cellulose fibrils (or nanofibrils) in cases where the cellulosic fibers (or nanofibers) consist of bundles of primary cellulose fibrils (or nanofibrils). While cellulose is a more rigid crystalline material, hemicellulose is amorphous and consequently has weaker mechanical properties. With its distribution throughout the structure of the cellulosic fibers (or nanofibers), and without wishing to be bound by theory, the inventors believe that hemicellulose may act to increase the flexibility of the nanocellulose, possibly acting as a plasticizer or toughening agent between the cellulose fibres and allowing individual cellulose fibres to creep and extend with respect to each other. While this might reduce the stiffness of the cellulosic fibers (or nanofibers), it can potentially increase the fracture toughness of the cellulosic fibers (or nanofibers) (indeed, when the present inventors homogenise base-treated spinifex, which is an example of a suitable source of cellulosic fibers (or nanofibers) for use in the present invention, and compare that to other softwood or hardwood pulps, the inventors have found that the spinifex can be exposed to a much higher degree of mechanical energy without fibril breakage, which appears to be an indication of a much higher degree of cellulose/nanocellulose toughness).

In some embodiments of the present invention, the hemicellulose content of the cellulosic fibers (from which the composition of the first aspect is formed) is at least 20% or 30 % by mass of the lignocellulosic components of the cellulosic fibers from which the composition of the first aspect is formed. Preferably, the hemicellulose content is from 20 to 55% w/w, or from 30% to 55 % w/w, or from 30 to 50% w/w, or from 36 to 48% w/w, or from 40 to 48% w/w or from 42 to 47% w/w, or any intermediate range within the ranges set out above, including 20-30% or 30-40% or 40-50% w/w. These high hemicellulose contents may be achieved by any means, including but not limited to, using plant feedstocks that are naturally high in hemicellulose and subsequent processing to produce cellulosic fibers (or nanofibers) that retain a high hemicellulose content, or alternatively, using a cellulosic material (or nanocellulosic material) that has lower hemicellulose content and mixing it with a separately produced hemicellulose material to give a mixture that provides a high hemicellulose content cellulose (or nanocellulose).

In some embodiments of the second and third aspects of the present invention, the hemicellulose content of the deep eutectic solvent treated cellulosic fibers (or the cellulosic nanofibers) is less than 20 % by mass of the lignocellulosic components of the cellulosic fibers. In one embodiment, the hemicellulose content of the deep eutectic solvent treated cellulosic fibers (or the cellulosic nanofibers) is less than 19%, 18%, 17%, 16%, 15%, 14%, 13%, 12%, 11%, 10%, 9%, 8%, 7%, 6%, 5%, 4%, 3% or 2% by mass of the lignocellulosic components of the cellulosic fibers; especially less than 10%, less than 6%, or less than 5%, or less than 4% w/w. In one embodiment, the cellulose content of the deep eutectic solvent treated cellulosic fibers (or the cellulosic nanofibers) is greater than 50% by mass of the lignocellulosic components of the cellulosic fibers. In one embodiment, the cellulose content of the deep eutectic solvent treated cellulosic fibers (or the cellulosic nanofibers) is less than 80%, 75%, 70%, 65%, 60% or 55% by mass of the lignocellulosic components of the cellulosic fibers; especially less than 65% or 55%. In one embodiment, the lignin content of the deep eutectic solvent treated cellulosic fibers (or the cellulosic nanofibers) is less than 20 % by mass of the lignocellulosic components of the cellulosic fibers. In one embodiment, the lignin content of the deep eutectic solvent treated cellulosic fibers (or the cellulosic nanofibers) is less than 19%, 18%, 17%, 16%, 15%, 14%, 13%, 12%, 11%, 10%, 9%, 8%, 7%, 6%, 5%, 4%, 3% or 2% by mass of the lignocellulosic components of the cellulosic fibers; especially less than 5% or less than 3%. Deep eutectic solvent treatment may reduce the amounts of lignocellulose, cellulose, hemicellulose and/or lignin in the cellulosic fibers.

In some embodiments the cellulosic fibers (or cellulosic material) is derived from a plant material having a hemicellulose content of 20% or 30% or higher (w/w). In other embodiments, the plant material has a hemicellulose content of from 20 to 55% w/w, or from 30 to 55% w/w, or from 30 to 50% w/w, or from 36 to 48% w/w, or from 40 to 48% w/w or from 42 to 47% w/w, or any intermediate range within the ranges set out above, including 20-30% or 30-40% or 40-50% w/w.

The cellulosic nanofibers may be of any suitable dimensions. As used herein, the term "nanofibers" means that the fibers are of a length that is typically below 1000 nm. The fibers are longer than they are wide. In one embodiment, the composition may comprise cellulosic nanofibers. In one embodiment, the nanofibers have a length of from about 50 nm to 1500 nm, or from about 50 nm to 1400 nm, or from about 100 nm to 1400 nm, or from about 200 nm to 1300 nm, or from about 300 nm to 1200 nm, or from about 400 nm to 1100 nm, or from about 500 nm to 1000 nm. In one embodiment, the cellulosic nanofibers have a width or diameter of from 0.5 nm to 20 nm, or from 1 nm to 15 nm, or from 1 nm to 10 nm, or from 1 nm to 6 nm, or from 2 nm to 5 nm, or about 3 nm. Without wishing to be bound by theory, the inventors advantageously believe that the cellulosic nanofibers may be long and thin, which help make the composition strong whilst still being very flexible. The cellulosic nanofibers may be substantially cylindrical. The cellulosic nanofibers may have a ratio of length to width (aspect ratio) of from 150:1 to 500:1, especially from 150:1 to 400:1. Spinifex grasses have the highest length to width ratio of cellulose fibers of any biomass reported. It will be appreciated that the fiber length and aspect ratio values of any given sample of cellulosic nanofiber will be composed by a distribution of values where the value quoted approximately represents an average of values for different fibers in a sample.

The cellulosic fibers (or nanofibers) may carry a charge. The cellulosic fibers may carry a positive or a negative charge, especially a negative charge.

In one embodiment, the cellulosic fibers (or nanofibers) are crosslinked. The cellulosic fibers (or nanofibers) may be crosslinked by a crosslinking agent. Suitable crosslinking agents may include compounds comprising a moiety including an epoxide, an alkene, an aldehyde, an imide, an amine a carboxylic acid, and an acrylamide. The crosslinking agent may include compounds comprising at least two moieties selected from the group consisting of: an epoxide, an alkene, an aldehyde, an imide, an amine a carboxylic acid, a urea and an acrylamide. In one embodiment, the crosslinking agent may be an amino acid. The cross linking agent may be selected from the group consisting of 1,4-butanediol diglycidyl ether, divinyl sulfone, 1,2,7,8-diepoxyoctane, hexamethylenediamine, glycine, epichlorohydrin, urea and methylenebisacrylamide. The crosslinking agent may be 1,4-butanediol diglycidyl ether, divinyl sulfone, 1,2,7,8-diepoxyoctane, glycine or urea. In one embodiment, the cellulosic nanofibers are not crosslinked, or are not treated with a crosslinked agent.

The composition may be homogenized or mechanically refined. The composition may be homogenized at a pressure of at least 400 bar, or at least 500 bar, 600 bar, 700 bar, 800 bar, 900 bar or 1000 bar. The composition may be homogenised at a pressure of about 1,100 bar. The composition may be mechanically refined by, for example, milling or ultrasonicating the composition. The composition may be homogenised multiple times, for example 1, 2, 3, 4, 5, 6, 7, 8, 9 or 10 times.

The cellulosic fiber (or cellulosic nanofiber, or DES cellulosic fiber or DES cellulosic nanofiber) may be present in the composition in any suitable concentration. In one embodiment the composition may comprise less than 10% w/v cellulosic fiber (or cellulosic nanofiber, or DES cellulosic fiber or DES cellulosic nanofiber), especially less than 9%, 8%, 7%, 6%, 5%, 4%, 3% or 2% w/v cellulosic fiber (or cellulosic nanofiber, or DES cellulosic fiber or DES cellulosic nanofiber). The composition may comprise from 0.01 % to 5% w/v cellulosic fiber (or cellulosic nanofiber, or DES cellulosic fiber or DES cellulosic nanofiber), or from 0.05% to 4% w/v, or from 0.1% to 2% w/v, or from 0.1% to 1.5% w/v, or from 0.1% to 1% w/v cellulosic fiber (or cellulosic nanofiber, or DES cellulosic fiber or DES cellulosic nanofiber). The composition may comprise 0.2%, 0.4%, 0.6%, 0.8%, 0.88% or 1.0% w/v cellulosic fiber (or cellulosic nanofiber, or DES cellulosic fiber or DES cellulosic nanofiber).

The composition may comprise any suitable solvent for injection. In one embodiment, the solvent is an aqueous solvent. The solvent may be saline, especially sterile saline. The solvent may be an aqueous buffer solution. The solvent may be Phosphate Buffered Saline (PBS). The aqueous buffer solution may be for maintaining the composition at close to physiological pH or at least within a range of about pH 6.0 to 9.0.

The composition may have any suitable pH for injection. In one embodiment, composition has a pH of from 4 to 9, especially from 5 to 8, or from 5.4 to 7.

The composition may be in any suitable form for injection. The composition would be in liquid or gel form, especially gel form. The composition may have any suitable viscosity. In one embodiment, the composition has a complex viscosity of 10 to 10,000 Pa.s at 1 rad/s and 25 °C, or from 50 to 5,000 Pa.s at 1 rad/s and 25 °C, or from 100 to 1,000 Pa.s at 1 rad/s and 25 °C. The composition may have a loss modulus of from 10 to 1,000 Pa at 1 rad/s and 25 °C, or from 10 to 500 Pa at 1 rad/s and 25 °C, or from 10 to 100 Pa at 1 rad/s and 25 °C.

The composition may have any suitable storage modulus (G'). In one embodiment, the composition has a storage modulus of from 10 to 10,000 Pa at a frequency of 0.1 to 1000 rad/s (or at 0.1, 1, 10 or 100 rad/s), or from 50 to 5,000 Pa at a frequency of 0.1 to 1000 rad/s (or at 0.1, 1, 10 or 100 rad/s), or from 100 to 1,000 Pa at a frequency of 0.1 to 1000 rad/s (or at 0.1, 1, 10 or 100 rad/s). In one embodiment, the composition has a storage modulus of from 10 to 10,000 Pa at a frequency of 0.1 to 1000 rad/s (or at 0.1, 1, 10 or 100 rad/s) at 25 °C, or from 50 to 5,000 Pa at a frequency of 0.1 to 1000 rad/s (or at 0.1, 1, 10 or 100 rad/s) at 25 °C, or from 100 to 1,000 Pa at a frequency of 0.1 to 1000 rad/s (or at 0.1, 1, 10 or 100 rad/s) at 25 °C.

In one embodiment, the composition may comprise a further agent. The further agent may be a rheology modifier. The rheology modifier may be, for example, hyaluronic acid, or crosslinked hyaluronic acid. The composition may also comprise an active agent. For example, the composition may comprise platelets (or platelet rich plasma). This may be advantageous for platelet-rich plasma therapy.

The composition may be formulated in unit dose form. For example, the composition may be presented in ampoules, pre-filled syringes, small volume infusions or in multi-dose containers. Such compositions may include a preservative.

In a fourth aspect, the present invention relates to a method of cosmetic treatment according to claim 1. The method comprises use of an injectable composition formed from cellulosic fibers and a deep eutectic solvent, wherein the injectable composition is obtainable by a preparation process comprising: (i) contacting cellulosic fibers with a deep eutectic solvent to provide deep eutectic solvent treated cellulosic fibers; (ii) washing the deep eutectic solvent treated cellulosic fibers; and (iii) homogenizing or mechanically refining the washed deep eutectic solvent treated cellulosic fibers to thereby provide the injectable composition.

In one embodiment, the composition comprises cellulosic nanofibers. In one embodiment, step (iii) provides the injectable composition comprising cellulosic nanofibers. Without wishing to be bound by theory, the inventors believe that homogenization or mechanically refining the composition may result in shear forces that disperse the fibers throughout the resultant composition, and which also may fragment or fibrillate the cellulosic fibers to thereby provide cellulosic nanofibers.

The method of the fourth aspect may produce the composition of the first, second or third aspects.

In one embodiment of the fourth aspect, the cellulosic fibers used in step (i) are bleached cellulosic fibers. Therefore, prior to step (i) the method may comprise the step of bleaching cellulosic fibers to provide bleached cellulosic fibers. As discussed above, suitable bleaching agents would be known to a skilled person. In one embodiment, the cellulosic fibers may be bleached a chlorite or hypochlorite, especially a chlorite, or sodium chlorite. The bleaching agent may be 1% w/v sodium chlorite aqueous solution. The cellulosic fibers may be bleached with any suitable concentration of bleaching agent and at any suitable temperature and any suitable pH. The ratio of cellulosic fibers to bleaching agent may be from 10:1 to 100:1, especially from 20:1 to 40:1 or about 30:1. The cellulosic fibers may be bleached at from 40 °C to 90 °C, or from 50 °C to 90 °C, or from 60 °C to 80 °C or about 70 °C. Before contacting the bleached cellulosic fibers with the deep eutectic solvent, the bleached cellulosic fibers may be washed before drying. The bleached cellulosic fibers may be washed with water at a temperature above 50 °C. The bleached cellulosic fibers may be washed until the pH of the pulp is greater than 6.5. The bleached cellulosic nanofibers may be dried at from 40 °C to 90 °C, or from 50 °C to 90 °C, or from 60 °C to 80 °C or about 70 °C.

The cellulosic fibers may be delignified prior to bleaching. Consequently, the cellulosic fibers used in the bleaching step may be delignified cellulosic fibers. In one embodiment, prior to bleaching the method may comprise the step of delignifying the cellulosic fibers. As bleaching may be optional, in one embodiment the cellulosic fibers used in step (i) are delignified cellulosic fibers. Therefore, prior to step (i) the method may comprise the step of delignifying cellulosic fibers to provide delignified cellulosic fibers. For the avoidance of doubt, the step of delignifying the cellulosic fibers means that there is a reduction in the amount of lignin in the cellulosic fibers; the cellulosic fibers may still comprise some lignin after this step. The delignification step may be performed with a base, especially a hydroxide, more especially sodium hydroxide. The sodium hydroxide may be 2 or 3% w/v sodium hydroxide. The delignification may be performed with any suitable concentration of delignifying agent (especially base) and at any suitable temperature. The ratio of cellulosic fibers to delignifying agent may be from 5:1 to 50:1, especially from 10:1 to 30:1 or about 20:1. The cellulosic fibers may be bleached at from 50 °C to 100 °C, or from 70 °C to 90 °C, or about 80 °C. Before contacting the delignified cellulosic fibers with the bleaching agent, the delignified cellulosic fibers may be washed. The delignified cellulosic fibers may be washed multiple times, for example at least 2 or 3 times. The delignified cellulosic fibers may be washed with an aqueous solvent, especially water. The aqueous solvent may be at any suitable temperature, especially from 30 °C to 90 °C, or from 40 °C to 80 °C, or from 50 °C to 70 °C or about 60 °C.

The cellulosic fibers may be suspended in a solvent prior to delignification. Consequently, the cellulosic fibers used in the delignification step may be soaked cellulosic fibers. In one embodiment, prior to delignification the cellulosic fibers may be suspended in a solvent. The solvent may be an aqueous solvent, especially water, or reverse osmosis water. The aqueous solvent may be at any suitable temperature. For example, the aqueous solvent may be at from 20 °C to 70 °C, or from 30 °C to 70 °C, or from 40 °C to 60 °C or about 50 °C. The cellulosic fibers may be suspended for any suitable length of time, for example at least 5 hours, at least 10 hours or at least 12 hours. The delignifying agent may be added to the soaked cellulosic fibers without filtering.

The cellulosic fibers may be ground prior to soaking. In one embodiment, prior to soaking the method may comprise the step of grinding cellulosic fibers to provide ground cellulosic fibers. The cellulosic fibers may be ground using a cutting mill. The cellulosic fibers may be passed through a mesh, especially a mesh of less than 5 mm, or a mesh of less than 3 mm or a mesh of 1 mm.

The cellulosic fibers may be mulched before grinding. Consequently, the cellulosic fibers used in the grinding step may be mulched cellulosic fibers. Therefore, prior to grinding the method may comprise the step of mulching the cellulosic fibers to provide mulched cellulosic fibers. After mulching the mulched cellulosic fibers may be washed. The mulched cellulosic fibers may be washed multiple times, for example at least 2 or three times. The washing may be performed with an aqueous solvent, especially water. The aqueous solvent may be at a temperature of from 50 °C to 100 °C, or from 70 °C to 90 °C, or about 80 °C. The mulched, washed cellulose fibers may be dried before grinding.

Therefore, in one embodiment in the preparation process of the cosmetic treatment of claim 1 , step (i) is preceded by: the step of delignifying cellulosic fibers; and optionally the step of bleaching the delignified cellulosic fibers.

In a further embodiment the method of claim 1 may comprise:
(i') mulching cellulosic fibers to provide mulched cellulosic fibers;
(ii') grinding the mulched cellulosic fibers to provide ground cellulosic fibers;
(iii') suspending the ground cellulosic fibers in a solvent to provide soaked cellulosic fibers;
(iv') delignifying the soaked cellulosic fibers to provide delignified cellulosic fibers;
(v') optionally bleaching the delignified cellulosic fibers to provide bleached cellulosic fibers;

(i) contacting the cellulosic fibers of step (iv') or step (v') with a deep eutectic solvent to provide deep eutectic solvent treated cellulosic fibers;
(ii) washing the deep eutectic solvent treated cellulosic fibers; and
(iii) homogenizing or mechanically refining the washed deep eutectic solvent treated cellulosic fibers to thereby provide the injectable composition.

The deep eutectic solvent used in the step of contacting the cellulosic fibers with a deep eutectic solvent may be as described above. This step may comprise the step of forming the deep eutectic solvent. The deep eutectic solvent may be formed by heating a Lewis acid and a Lewis base. The heating may be at a temperature of from 50 °C to 100 °C, or from 70 °C to 90 °C, or about 80 °C. The molar ratio of cellulosic fibers to deep eutectic solvent may be from 1:2 to 1:50, or from 1:2 to 1:40, or from 1:2 to 1:30, or from 1:5 to 1:20 or about 1:10. The step of contacting the cellulosic fibers with a deep eutectic solvent may be performed at any suitable temperature, such as from 100 °C to 250 °C, or from 100 °C to 200 °C, or from 120 °C to 180 °C, or from 130 °C to 170 °C, or from 140 °C to 160 °C, or about 150 °C. The step of contacting may be performed for any suitable length of time, for example from 10 minutes to 2 hours, especially about 30 minutes.

The step of washing the deep eutectic solvent treated cellulosic fibers may be performed any suitable number of times. In one embodiment, the deep eutectic solvent treated cellulosic fibers are washed at least 2, 3, 4, 5, 6, 7 or 8 times. The washing may be performed with an aqueous solvent, especially water, more especially purified water. The washing may be performed with an aqueous solvent at any suitable temperature. For example, the solvent may be at from freezing (0 °C) to boiling (100 °C). In one embodiment, the washing is performed with solvents at at least two different temperatures. The washing may be performed with a first solvent at from 50 °C to 100 °C, especially from 70 °C to 100 °C, or from 80 °C to 100 °C, or from 90 °C to 100 °C. The washing may be performed with a second solvent at from 0 °C to 50 °C, or from 0 °C to 30 °C, or from 0 °C to 20 °C, or from 0 °C to 10 °C. The washing may be performed with the first solvent at least one, two, three or four times. The washing may be performed with the second solvent at least one, two, three or four times.

The step of washing may comprise exchanging the solvent of the deep eutectic solvent treated cellulosic fibers. The solvent may be exchanged with saline (especially sterile saline), or an aqueous buffer solution (such as Phosphate Buffered Saline (PBS)).

The method may comprise the step of diluting the washed deep eutectic solvent treated cellulosic fibers (or the solvent exchanged fibers) to a desired concentration. The desired concentration may be less than 10% w/v deep eutectic solvent treated cellulosic fibers, especially less than 9%, 8%, 7%, 6%, 5%, 4%, 3% or 2% w/v deep eutectic solvent treated cellulosic fibers. The desired concentration may be from 0.01 % to 5% w/v deep eutectic solvent treated cellulosic fibers, or from 0.05% to 4% w/v, or from 0.1% to 2% w/v, or from 0.1% to 1.5% w/v, or from 0.1% to 1% w/v deep eutectic solvent treated cellulosic fibers. The desired concentration may be 0.2%, 0.4%, 0.6%, 0.8%, 0.88% or 1.0% w/v deep eutectic solvent treated cellulosic fibers.

In one embodiment, the deep eutectic solvent treated cellulosic fibers may be washed with an acid prior to homogenization or mechanically refining. The acid wash may be a mild acid wash. The acid may be, for example, sulphuric or phosphoric acid.

The step of homogenizing the washed deep eutectic solvent treated cellulosic fibers may be at any suitable pressure, but may especially be at high pressure. The pressure may be at least 400 bar, or at least 500 bar, 600 bar, 700 bar, 800 bar, 900 bar or 1000 bar. The pressure may be about 1,100 bar. The step of mechanically refining the washed deep eutectic solvent treated cellulosic fibers may be or comprise milling or ultrasonicating the washed deep eutectic solvent treated cellulosic fibers.

Features of the fourth aspect of the present invention may be as described for the first, second or third aspect.

In a fifth aspect, the present invention provides a cosmetically acceptable injectable composition prepared by the preparation process as defined in any one of claims 1 to 6.

In a sixth aspect, the present invention relates to the cosmetic use of the composition of the first, second, third or fifth aspect as a dermal filler.

In a seventh aspect, the invention relates to an injectable composition of the first, second, third or fifth aspect formed from cellulosic fibers and deep eutectic solvent for use in plasma therapy.

As used herein, the terms "treatment" (or "treating") and "prevention" (or "preventing") are to be considered in their broadest contexts. For example, the term "treatment" does not necessarily imply that a patient is treated until full recovery. The term "treatment" includes amelioration of the symptoms of a disease, disorder or condition, or reducing the severity of a disease, disorder or condition. Similarly, "prevention" does not necessarily imply that a subject will never contract a disease, disorder or condition. "Prevention" may be considered as reducing the likelihood of onset of a disease, disorder or condition, or preventing or otherwise reducing the risk of developing a disease, disorder or condition.

As used herein, the terms "subject" or "individual" or "patient" may refer to any subject, particularly a vertebrate subject, and even more particularly a mammalian subject, for whom therapy is desired. Suitable vertebrate animals include, but are not restricted to, primates, avians, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes). A preferred subject is a human.

In a eigth aspect, the present invention relates to a cosmetic method of increasing volume in or under the skin of a subject, comprising injecting the composition of the first, second, third or fifth aspect in or under the skin of the subject.

The composition may be injected in any suitable way. For example, the composition may be injected subcutaneously, intradermally or intramuscularly; especially subcutaneously or intradermally. The composition may be injected into the face or breasts of a subject. The composition may be injected to increase the volume of the lip, breast or cheek of the subject. The method may provide a face lift for the subject. The method may ameliorate the appearance of nasolabial folds or perioral rhytids. The method may ameliorate the appearance of wrinkles in the skin of the subject.

In embodiments of the sixth to eight aspects of the present invention, the composition may be administered in an effective amount. As used herein, "effective amount" refers to the administration of an amount of the composition sufficient to at least partially attain the desired response, or to achieve the desired effect. It is expected that the "effective amount" will fall within a broad range that can be determined through routine trials. Decisions on dosage and the like would be within the skill of the medical practitioner or veterinarian responsible for the care of the patient.

Embodiments of the seventh aspects of the present invention refer to platelet rich plasma therapy. In these embodiments, the composition being administered would comprise platelets, especially platelet rich plasma. Platelet rich plasma is administered by injection. Such therapy may assist in wound healing, or in treating injuries (especially to tendons and/or ligaments).

Features of the sixth to eigth aspects of the present invention may be as described for the first to fifth aspects of the present invention.

Unless defined otherwise, all technical and scientific terms used herein have the same meaning as would be commonly understood by those of ordinary skill in the art to which this invention belongs.

Reference throughout this specification to 'one embodiment' or 'an embodiment' means that a particular feature, structure, or characteristic described in connection with the embodiment is included in at least one embodiment of the present invention. Thus, the appearance of the phrases 'in one embodiment' or 'in an embodiment' in various places throughout this specification are not necessarily all referring to the same embodiment.

In the present specification and claims, the word 'comprising' and its derivatives including 'comprises' and 'comprise' include each of the stated integers but does not exclude the inclusion of one or more further integers.

### BRIEF DESCRIPTION OF DRAWINGS

Examples of the invention will now be described by way of example with reference to the accompanying Figures, in which:
Figure 1 illustrates a process for producing compositions according to the present invention;
Figures 2A and 2B are graphs illustrating the rheology of compositions according to the present invention, in which the gels were prepared with sterile saline. Figure 2A is a graph illustrating how the complex modulus varies with respect to frequency for the samples (the lower graph is a black and white version of the upper graph), and Figure 2B is a graph illustrating how the complex viscosity varies with respect to frequency for the samples (the lower graph is a black and white version of the upper graph);

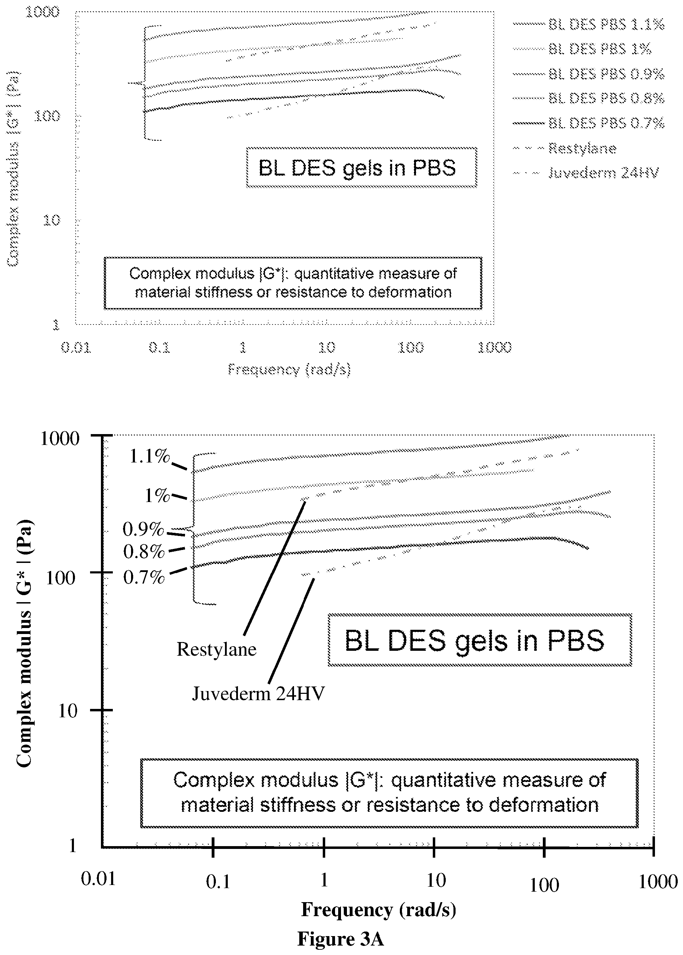

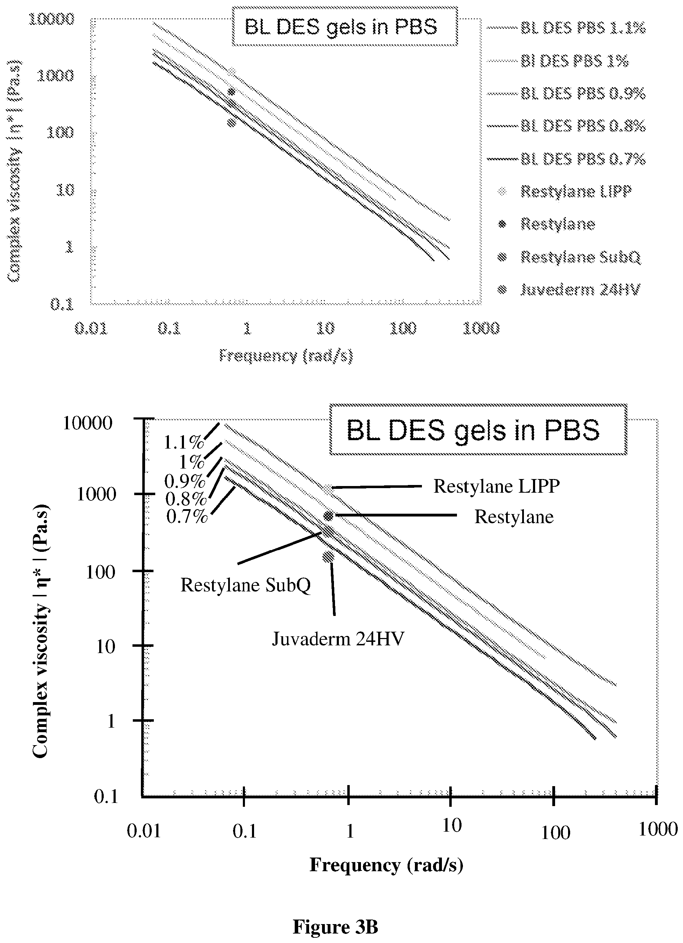

Figures 3A and 3B are graphs illustrating the rheology of compositions according to the present invention, in which the gels were prepared with Phosphate Buffered Saline (PBS). Figure 3A is a graph illustrating how the complex modulus varies with respect to frequency for the samples (the lower graph is a black and white version of the upper graph), and Figure 3B is a graph illustrating how the complex viscosity varies with respect to frequency for the samples (the lower graph is a black and white version of the upper graph). Figures 3A and 3B illustrate this rheology compared to the published rheology of commercial dermal fillers;
Figures 4A and 4B are graphs illustrating the rheology of compositions according to the present invention, as compared to Restylane^{™} commercial dermal filler samples. Figure 4A is a graph illustrating how the storage modulus and loss modulus varies with respect to frequency for the samples (the lower graph is a black and white version of the upper graph), and Figure 4B is a graph illustrating how the complex viscosity varies with respect to frequency for the samples (the lower graph is a black and white version of the upper graph);
Figures 5A and 5B are graphs illustrating the rheology of compositions according to the present invention, as compared to Juvéderm^{™} commercial dermal filler samples. Figure 5A is a graph illustrating how the storage modulus and loss modulus varies with respect to frequency for the samples (the lower graph is a black and white version of the upper graph), and Figure 5B is a graph illustrating how the complex viscosity varies with respect to frequency for the samples (the lower graph is a black and white version of the upper graph);
Figures 6A and 6B are graphs relating to the injectability of compositions according to the present invention. Figure 6A is a graph illustrating the force required to displace various compositions of the invention (the lower graph is a black and white version of the upper graph). Figure 6B is a graph illustrating the glide force for the compositions with 30 and 31 gauge needles (the lower graph is a black and white version of the upper graph);
Figure 7 is a graph (full view and expanded view) illustrating the force required to inject a composition according to the present invention (0.88% w/v DES CNF), compared to commercial dermal filler samples (the lower graph is a black and white version of the upper graph);
Figure 8 is a graph illustrating how the rheology of a gel according to the present invention (0.8% w/v DES CNF) changes over time when stored for up to 90 days at 4 °C (the lower graph is a black and white version of the upper graph);
Figure 9 is a graph illustrating how the rheology of a composition according to the present invention (0.8% w/v DES CNF) changes over time when stored for up to 90 days at 23 °C (the lower graph is a black and white version of the upper graph);
Figure 10 is a graph illustrating how the rheology of a composition according to the present invention (0.8% w/v DES CNF) changes over time when stored for up to 90 days at 55 °C (the lower graph is a black and white version of the upper graph);
Figure 11 is a graph illustrating how the rheology of a composition according to the present invention (0.8% w/v DES CNF) changes over time when stored for up to 90 days at 37 °C in 5% CO₂ (the lower graph is a black and white version of the upper graph);
Figures 12A and 12B are graphs illustrating how the rheology (at 0.1 Hz) of compositions according to the present invention changes over 60 days when stored at 55 °C, as compared to commercial dermal filler samples. Figure 12A illustrates storage modulus (the lower graph is a black and white version of the upper graph), and Figure 12B illustrates the loss modulus over this period (the lower graph is a black and white version of the upper graph); and
Figure 13 is a Transmission Electron Microscopy image of a freeze-dried sample of a homogenized deep eutectic solvent treated cellulosic nanofiber.

Preferred features, embodiments and variations of the invention may be discerned from the following Examples which provides sufficient information for those skilled in the art to perform the invention. The following Examples are not to be regarded as limiting the scope of the preceding Summary of the Invention in any way.

### EXAMPLES

### Example 1: Preparation of Cellulose Nanofibres

Spinifex grass *(Triodia pungens)* was collected from Camooweal in Queensland, Australia.

Grass was pre-screened and the leaves were selected and cut-off from the woody stem. This pre-screened material is referred to as the "tips". The tips were then mulched, washed 6 times with water at 80 °C for 1 hour, air dried and ground to a fine powder using a Retsch cutting mill with a 1 mm mesh.

The ground washed grass was soaked overnight in reverse osmosis (RO) water at 50 °C (with a water to grass ratio of 20:1), then treated with 3% (w/v) sodium hydroxide at 80 °C for two hours. The resulting pulp was washed with hot water (60 °C) three times to remove dissolved material. This step delignifies the cellulosic nanofibers.

The alkali pulp was then bleached twice using 1% (w/v) sodium chlorite aqueous solution at 70 °C for one hour with a 30:1 solvent to grass ratio at pH 4 (with addition of glacial acetic acid). After this time, the mixture was poured into a sieve, and boiling deionised water was slowly poured over the pulp in the sieve, turning over the pulp to evenly wash it. Following this, water at 55 °C was poured over the pulp in the sieve. The pulp was then transferred to a beaker and water was added and the bleaching process repeated as described. After the bleached pulp was washed as previously described, the pulp was washed with further hot water (at 55 °C) and boiling deionised water. On confirmation that the pH of the pulp is greater than 6.5 the pulp was transferred from the sieve and dried at 70 °C for 18 hours in a convection oven.

The components of Deep Eutectic Solvent (DES) treatment (sulfamic acid:urea at a molar ratio of 1:3) were mixed together over an oil bath at 80 °C until a clear solution was obtained (around two hours). Dried bleached cellulose pulp (at a cellulose:sulfamic acid molar ratio of 1:10) was immersed in DES. Then, the temperature of the oil bath was increased to 150°C and the reaction allowed to proceed for 30 minutes. The reaction was terminated by the addition of excess of water, followed by intensive centrifuging and washing (4 cycles with boiled MilliQ^{™} purified water and 4 cycles with cold MilliQ^{™} purified water). Small aliquots of supernatant were collected from the centrifuged gel batch after each washing step for (a) visual observation (colour, cloudiness), and (b) HPLC-MS analysis (urea) and LC-MS (sulfamic acid) if necessary to detect residual levels of urea, sulfamic acid, and any other potential by-products (such glucose and its glucose derivatives of sulfamic acid and urea, sulfamic acid derivatives of urea and N-substituted urea, although it was expected that these were very unlikely at the 1:3 sulfamic acid:urea ratio).

The washed DES treated cellulose fibres were centrifuged and diluted with a cold Phosphate Buffered Saline (PBS) (although sterile saline may be used instead) to wash the cellulose in four cycles of washing and centrifuging. Small aliquots of supernatant were collected from the centrifuged gel batch after each washing step for (a) visual observation (colour, cloudiness), and (b) analysis, as above, to detect residual levels of urea and sulfamic acid.

The washed cellulose in PBS was diluted to a concentration of 1 wt% in MilliQ^{™} purified water or 2 wt% in PBS (or sterile saline) and then passed through a high pressure homogeniser (GEA, PandaPlus 2000) several times as follows: one pass at 400 bar, one pass at 700 bar and 3 passes at 1100 bar.

The residual level of sulfamic acid in washing supernatants as outlined above, was evaluated by LC-MS, and the residual level of urea was evaluated by HPLC-MS (Invitrogen urea assay). The results are provided in Tables 1 and 2. For Table 2, the urea concentration in the PBS control was below the lower detection limit. Furthermore, around 7-20 mg/dL urea is considered normal in human blood.

**Table 1: Sulfamic acid concentrations (mg/L) in wash supernatants**

| Sulfamic acid concentration (mg/L) | Washed hot MilliQ^{™} water | Washed cold MilliQ^{™} water | Washed PBS |
|---|---|---|---|
| 1^{st} wash | 46,317 ± 272 | 114 ± 1 | 0.226 ± <0.01 |
| 2^{nd} wash | 13,294 ± 241 | 18.1 ± 0.1 | <0.01 ± <0.002 |
| 3^{rd} wash | 2,173 ± 12.4 | 1.49 ± 0.02 | <0.01 ± <0.002 |
| 4^{th} wash | 438.13 ± 48.6 | 0.12 ± <0.002 | <0.01 ± <0.002 |

**Table 2: Urea concentrations (mg/L) in wash supernatants**

| Urea concentration (mg/dL) | Washed hot MilliQ^{™} water | Washed cold MilliQ^{™} water | Washed PBS |
|---|---|---|---|
| 1^{st} wash | n/a (over upper detection limit (UDL)) | 2.573 ± 0.060 | n/a (below lower detection limit (LDL)) |
| 2^{nd} wash | n/a (over UDL) | 0.917 ± 0.019 | n/a (below LDL) |
| 3^{rd} wash | 16.983 ± 3.480 | 0.284 ± 0.018 | n/a (below LDL) |
| 4^{th} wash | 11.679 ± 0.116 | 0.024 ± 0.010 | n/a (below LDL) |

From the above prepared single batch of DES Cellulose NanoFibre (CNF) gel, a series of dilutions were prepared for rheological and injectability studies. Gels were prepared with DES CNF at a concentrations including 0.88%, 0.8%, 0.7%, 0.6%, 0.4% (w/v).

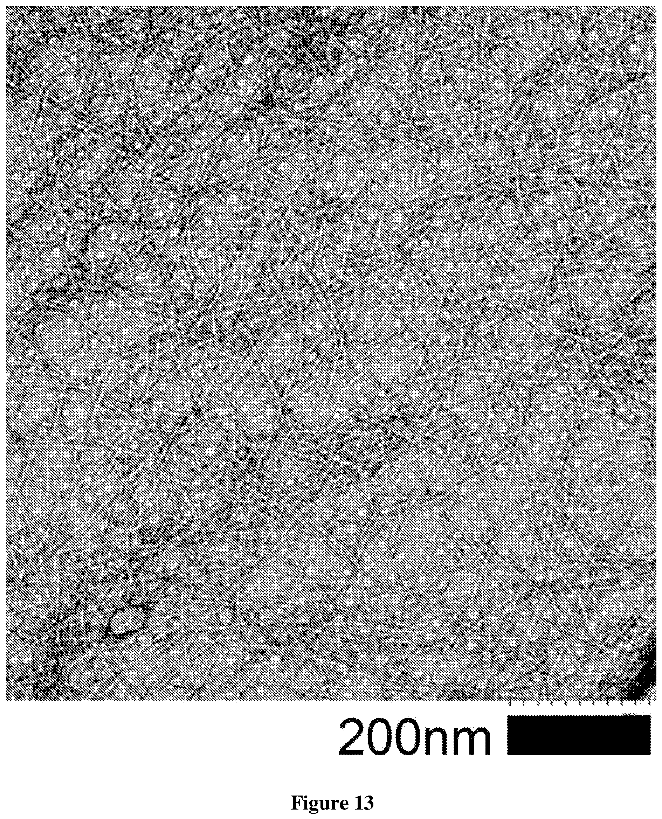

DES CNF gels were also prepared in saline solution, rather than in PBS. The procedure to prepare these gels were the same as those outlined above, except that final centrifuging and washing was done with saline solution (0.9%wt NaCl) (4 cycles with cold saline). Small aliquots of supernatant were collected from the centrifuged gel batch after each washing step for (a) visual observation (colour, cloudiness), and (b) analysis, as above, to detect residual levels of urea and sulfamic acid. Furthermore, DES cellulose was diluted to a concentration of 1.5wt% in saline and then passed through a high pressure homogeniser (GEA, PandaPlus 2000) several times as follows: one pass at 400 bar, one pass at 700 bar and 3 passes at 1100 bar. Gels were prepared with DES CNF at concentrations including 1.1%, 1%, 0.9%, 0.8% and 0.7% (w/v). A Transmission Electron Microscopy image of homogenised deep eutectic solvent treated cellulosic nanofiber in MilliQ^{™} water is provided in Figure 13.

The dry mass of CNF in prepared gel after completing washing with hot MilliQ^{™} water (4 times) and cold MilliQ^{™} water (4 times) was measured by Mettler Toledo moisture content analyser (HX204 Moisture analyser). To calculate the amount of salt after solvent exchanging with PBS buffer, the dry mass of CNF+PBS salts was also measured by Mettler Toledo moisture content analyser.

The ratio of CNF to salts (e.g. from PBS), was determined by thermogravimetric analysis (TGA) of dried gel. This was to also enable accurate normalisation of gel formulations with respect to true and accurate spinifex CNF content (w/v %) for subsequent rheology and injectability measurements.

The above steps were performed with high purity reagents, where applicable.

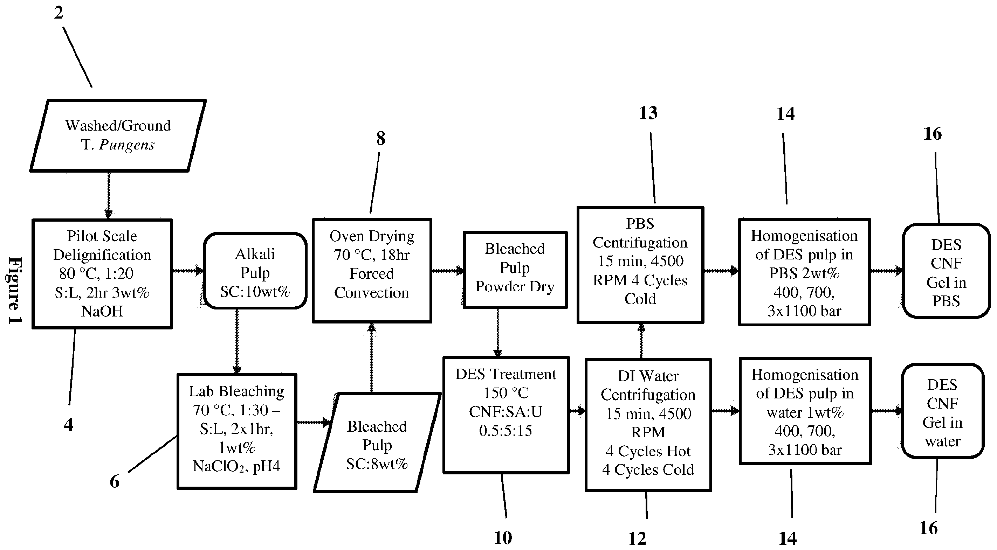

A very similar process is illustrated in Figure 1. In Figure 1 ground and washed cellulosic nanofibers is illustrated at **2,** and these are delignified at **4.** The nanofibers are bleached at **6,** then dried at **8,** before being contacted with a deep eutectic solvent at **10.** The cellulosic nanofibers complexed with a deep eutectic solvent were washed at **12,** and then homogenized at **14** to thereby provide the injectable composition **16.** The washing step **12** may also include solvent exchange step **13.**

### Example 2: Quality Control Assessment

For residual metal contaminants, samples were sent for Inductively Coupled Plasma - Optical Emission Spectrometry (ICP-OES) after each-and-every step of processing. Results are provided in Table 3 below.

**Table 3: ICP-OES Results (ppm)**

| | DES (water) CNF | Comment |
|---|---|---|
| Hg, Sr | ≤ 0.001 | |
| As, Ba, Co, Pb, Mo | ≤ 0.007 | |
| Al, Cd, Cr, Mn, Ni | ≤ 0.03 | In water, Al ~ 0.03, Mn ~0.01 |
| Cu | 0.052 | |
| Fe | 0.100 | |
| Zn | 0.126 | |
| Ca | 0.327 | In water, Ca is ~ 0.05 |
| B | 0.809 | |
| Si | 16.028 | There are SiO₂ phytoliths in grass |
| K | 0.034 | In water, K is ~ 0.01 |
| Na | 1.034 | In water, Na is ~1 |
| S | 67.902 | DES treatment |

X-Ray Photoelectron Spectroscopy (XPS) surface analysis on the pulp before DES treatment and the pulp after DES treatment, showed that N and S were present only after DES treatment. This indicates DES sulfation.

### Example 3: Rheology of DES gels and commercial dermal fillers

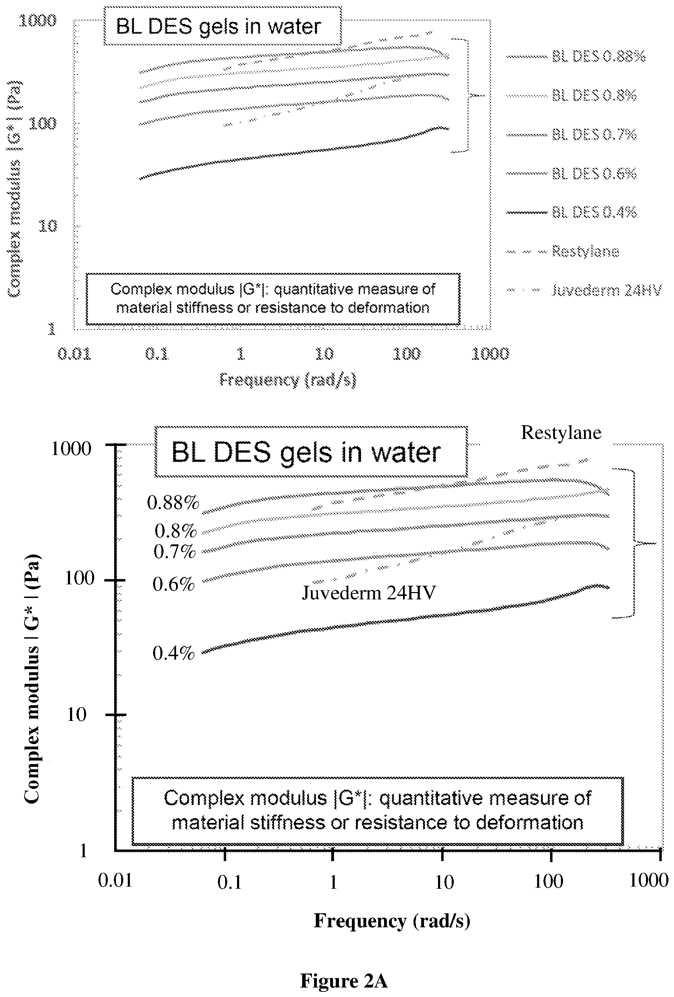

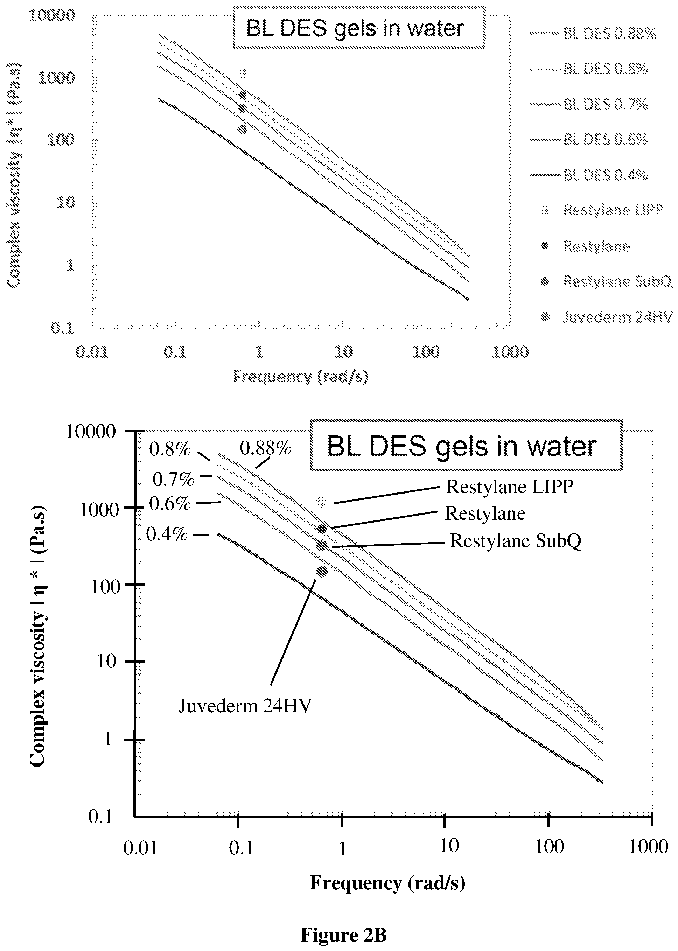

The gels prepared in the preceding experiments were tested for their rheological properties, and the results were compared with the published results for the hyaluronic acid (HA) dermal fillers Restylane^{™}, Restylane^{™} LIPP, Restylane^{™} SubQ and Juvéderm^{™} 24HV (Falcone, S.J. and R.A. Berg, Crosslinked hyaluronic acid dermal fillers: A comparison of rheological properties. Journal of Biomedical Materials Research Part A, 2008. 87A(1): p. 264-271). The results are illustrated in Figures 2 and 3. In Figures 2 and 3, BL DES represents a gel prepared according to Example 1. BL DES gels are those prepared with sterile saline (Figure 2). BL DES PBS gels are those prepared with PBS (Figure 3).

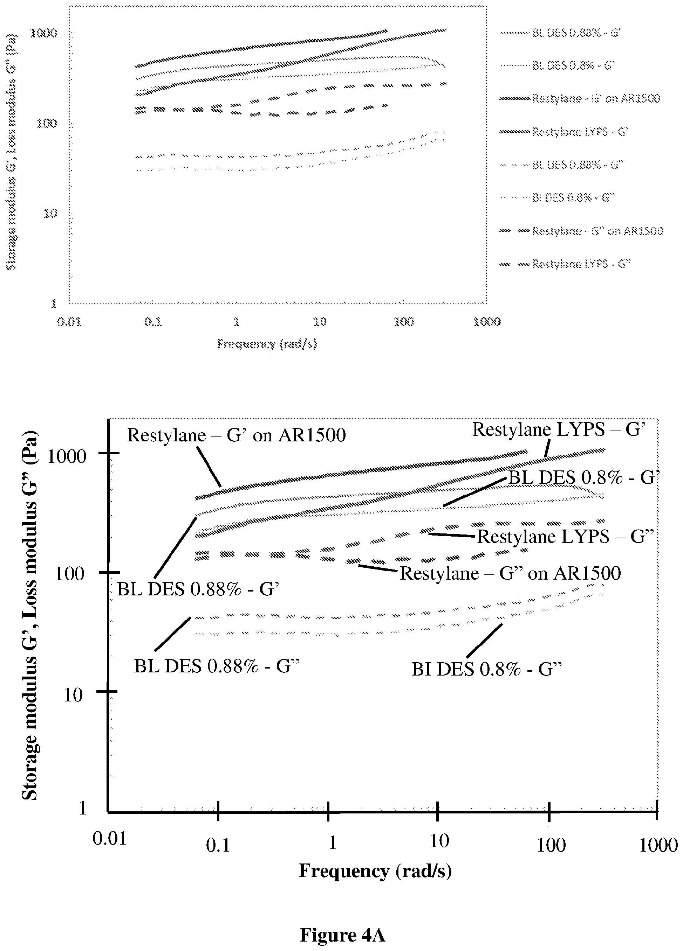

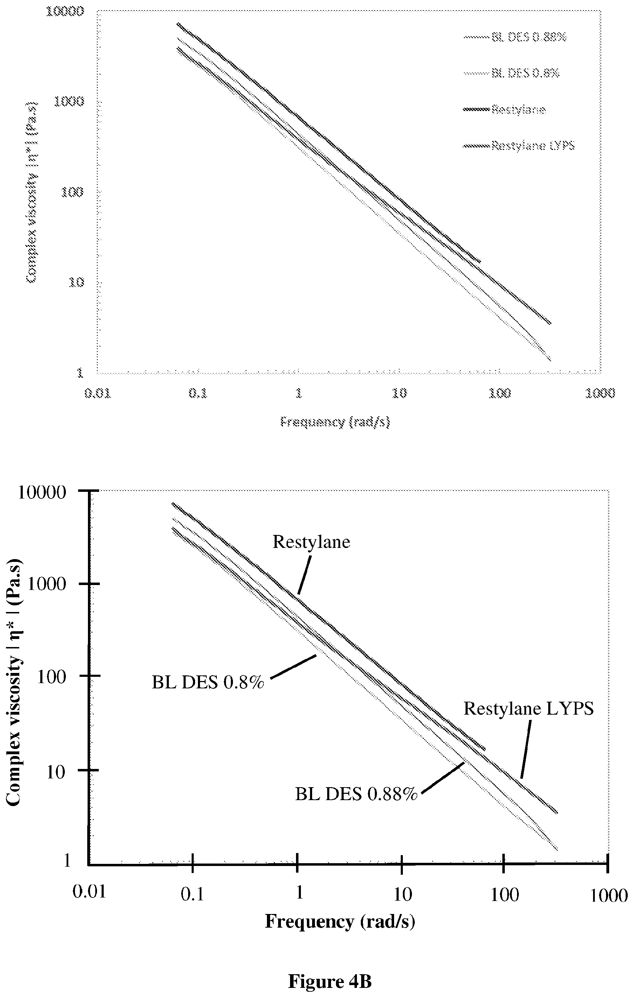

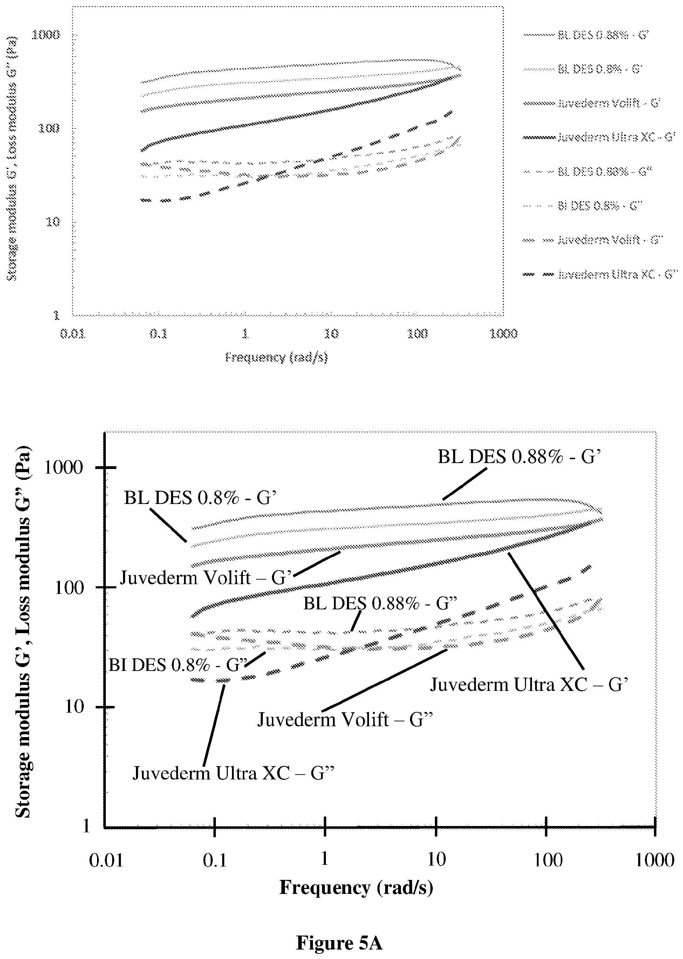

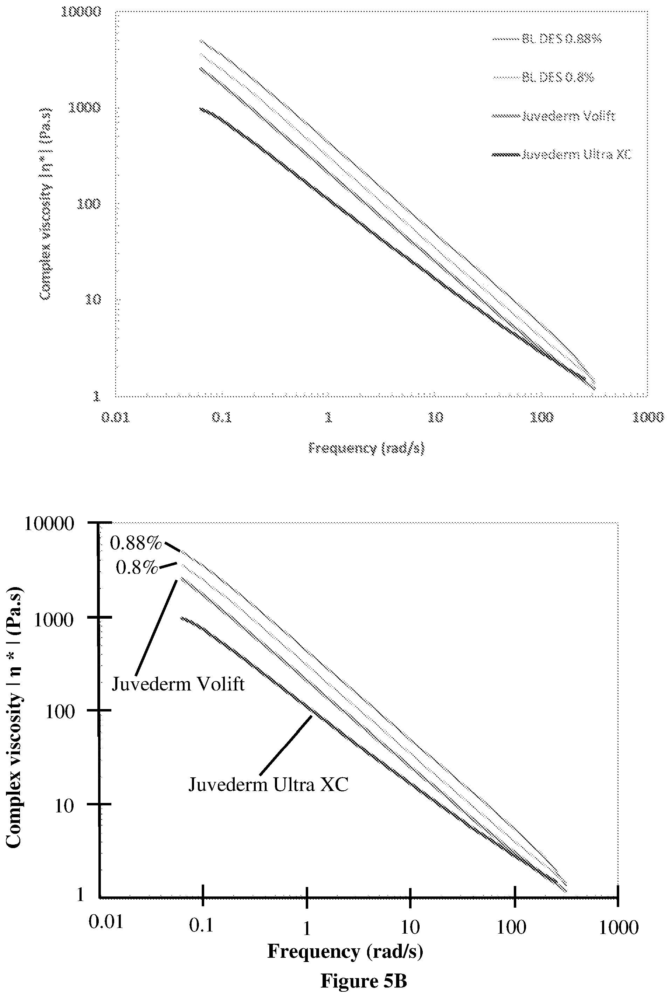

Figures 4 and 5 illustrate the rheology of gels prepared according to Example 1, as compared to actual commercial dermal filler samples. The commercial dermal filler samples include: Figures 4A and 4B - Restylane^{™} and Restylane^{™} LYPS (gels with 20 mg/mL hyaluronic acid, and with lidocaine); and Figures 5A and 5B - Juvéderm^{™} Volift (gel with 17.5 mg/mL hyaluronic acid, and with lidocaine) and Juvéderm^{™} Ultra XC (gel with 24 mg/mL hyaluronic acid, and with lidocaine). By comparison in Figures 4 and 5 the gels of Figure 1 are at 6-10 mg/mL CNF.

In Figures 2-5, dynamic rheological measurements were carried out on a TA Instruments rheometer (AR1500) fitted with a cone and plate geometry (40mm diameter). All measurements were performed at 25°C. Dynamic measurements were made over a frequency range of 0.1 to 100Hz. The experiments were performed in triplicate.

### Example 4: Injectability of DES gels and commercial dermal fillers

The measurement of the injection force required to push a gel through a 30G needle was performed in compression mode on an Instron mechanical tester fitted with a 500N load cell. A 1 mL syringe filled with 1 mL of gel formulation (with no air bubbles) and fitted with a 30G or 31G needle was positioned in a holder, directed downwardly. The plunger end of the needle was placed in contact with the load cell assembly. Testing was carried out at a crosshead speed of 1mm/s, which is representative of manual syringe delivery to patients. The loading force required to displace the plunger was measured at a function of displacement. The following parameters were determined:
- Initial glide force / Plunger break loose force: force required to initiate movement of the plunger;
- Maximum force: highest force measured before the plunger finishes its course;
- Dynamic glide force: the force required to sustain the movement of the plunger to expel the content of the syringe.

The force values were normalized by the cross sectional area of the cylindrical plunger. The experiments were performed in triplicate.

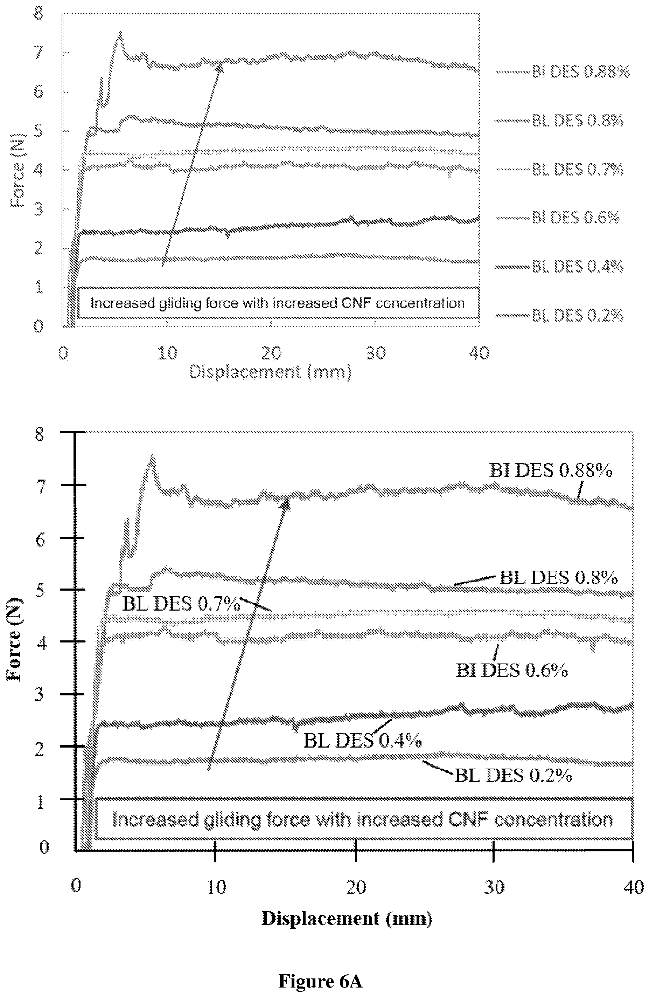

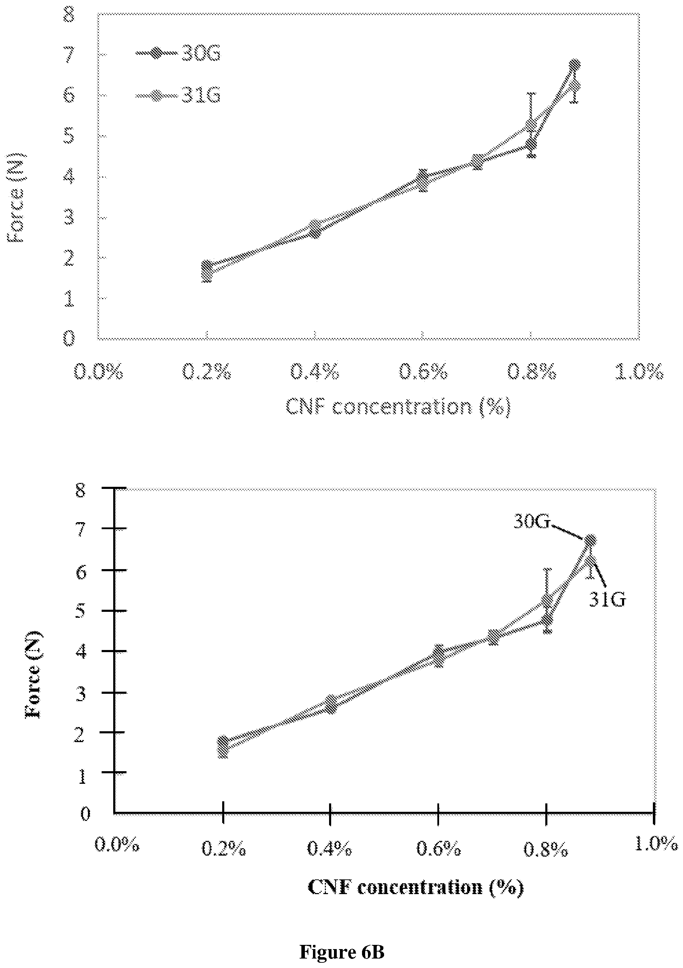

The results are illustrated in Figures 6A and 6B. Figure 6A shows the force required to displace various gels of Example 1. Figure 6B shows the glide force for DES gels (in saline) with 30 and 31 gauge needles. As illustrated in Figure 6B the force required for a 30 and a 31 gauge needle is extremely similar.

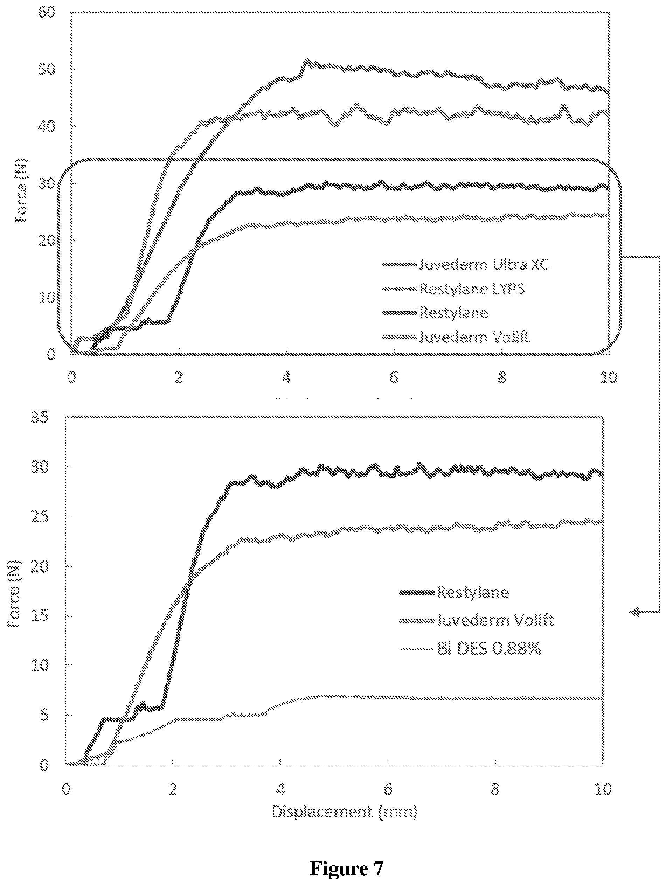

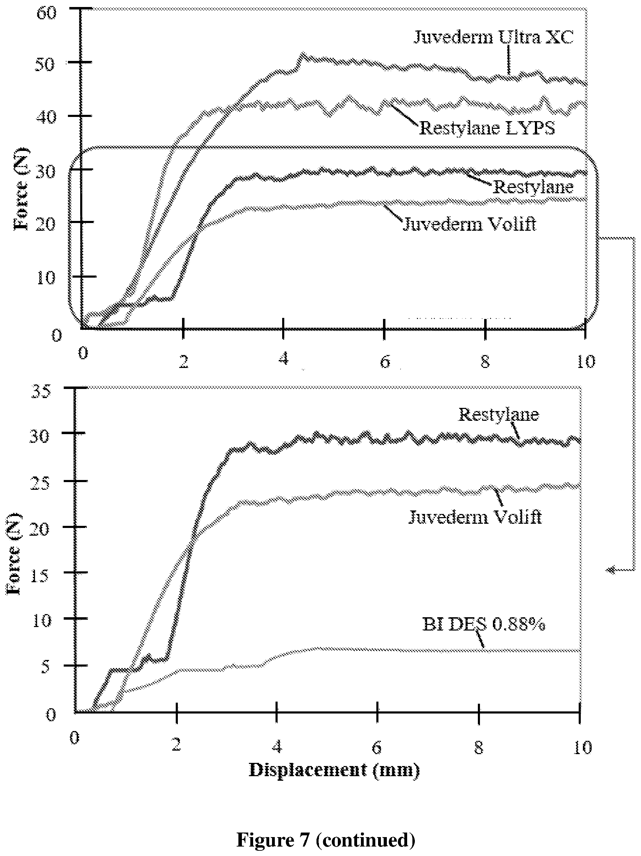

When compared with Restylane^{™}, Restylane^{™} LYPS, Juvéderm^{™} Ultra XC and Juvéderm^{™} Volift, the force required to inject the 0.88% w/v DES CNF gel of Example 1 is significantly smaller. This is illustrated in Figure 7. Restylane includes ~ 250 micron particles of crosslinked hyaluronic acid. The gels of Example 1 comprise ~500-1000 nm long x ~3 nm wide rods.

### Example 5: Cell Proliferation / Cytotoxicity Analysis

MilliQ^{™} purified water washed Gel (no PBS) was prepared and supplied in sterile containers at two starting concentrations (0.8% and 0.4% (w/v)). For cytotoxicity assays, a fibroblast (3T3) cell line was seeded in 96-well plates @ 2.5-5x103 cells/well in DMEM. CNF gels were diluted as-follows and sterilised via microwave treatment (60% power for 5 seconds until just boiling).

The 0.5% final gel conc. (3.2 mL final volume) included:
- 0.8% gel solution - 2.0 mL
- 10x Dulbecco's Modified Eagle Medium (DMEM) - 0.32 mL
- Fetal Calf Serum (FCS) - 0.32 mL
- Distilled water (DW) - 1.2 mL

For the 0.1% final gel conc. (4.0 mL final volume)
- 0.4% gel solution 1.0 mL
- 10x DMEM 0.4 mL
- FCS 0.4 mL
- DW 2.2 mL

Plate 1 - Cell proliferation was determined by MTT assay. Gels at 0% (medium alone), 0.1% and 0.5% were added to wells and incubated for 24 or 48h. 0.01 mL MTT reagent (Sigma Aldrich) was added for 8h, then SDS reagent overnight at 37°C (5% CO₂). Absorbance of formazan product read in Tecan plate reader (at 570nm, according to manufacturer's instructions).

Plate 2 - 3T3 cells were seeded into black with clear bottom 96-well plate @ ~5x10³ cells/well in DMEM + 10% FCS. Cellulose gel (or medium control) was added to wells. Medium was removed, washed x 1 with Phenol Red free Optimem medium, then added PI/Hoechst 33342 dye 1/1000 in Optimem for 1h before reading fluorescence on Tecan plate reader.

Plate 3 - CNF gels (0.5%, 0.1%) were added to wells of a 96 well plate (0.1 mL/well) and allowed to set overnight. 3T3 cells were this time seeded on top of the CNF gels as well as the empty well tissue culture plastic (~5x10³ cells/well in DMEM + 10% FCS) and incubated for 5d, followed by optical microscopy at two magnifications (x10, x20).

Testing illustrated that the cellulose gels of Example 1 did not kill the cells, and that cells do not proliferate on the gel. While the cells did not grow the same way in the presence of the cellulose gels as in its absence, cells grown in the presence of the cellulose gels were viable.

### Example 6: Gel aging of DES gels and commercial dermal fillers

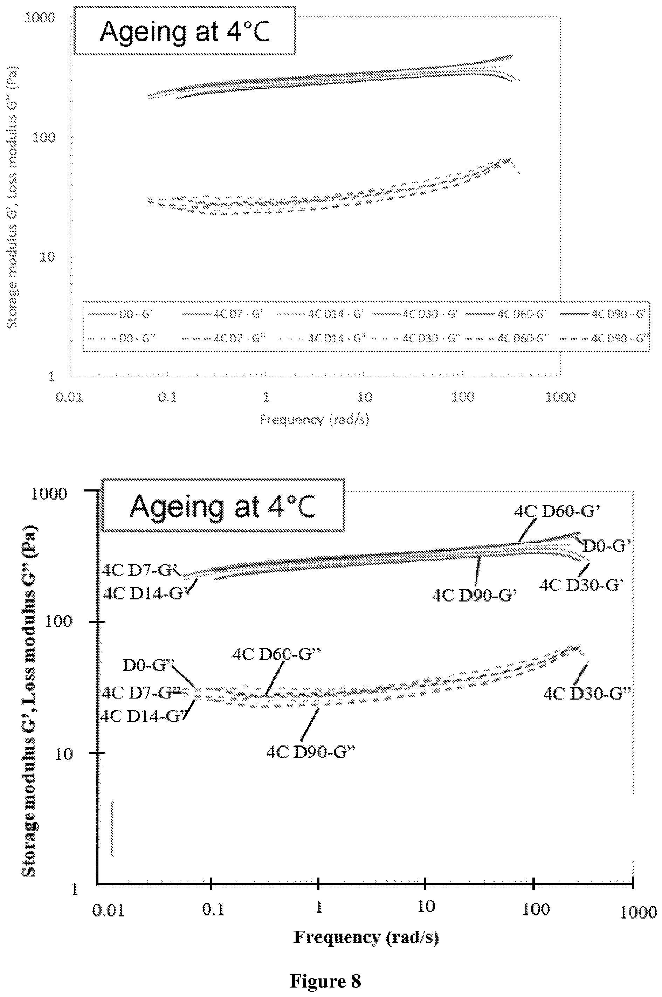

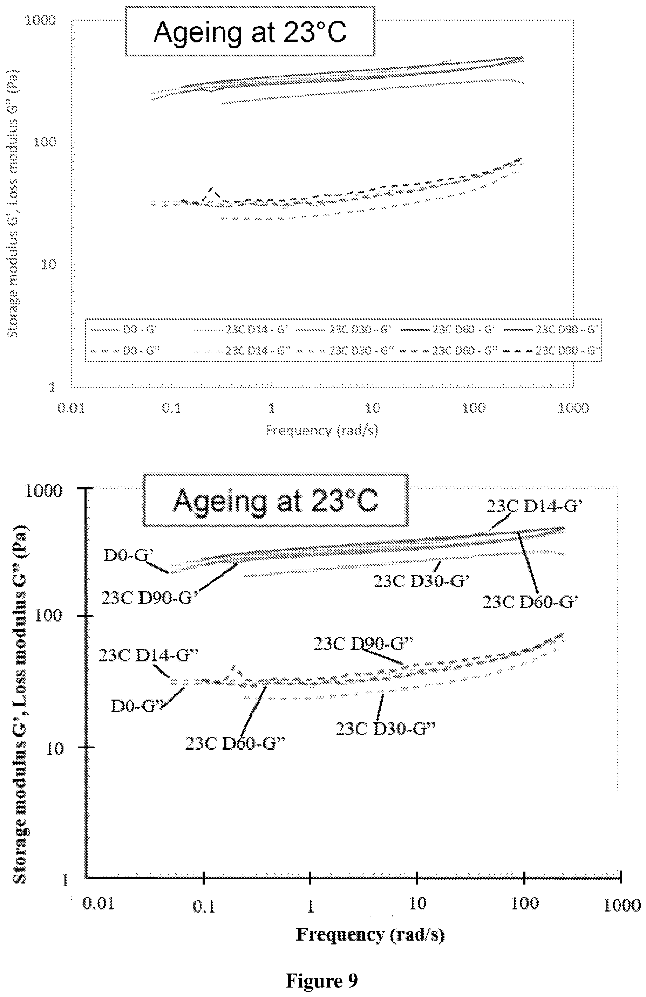

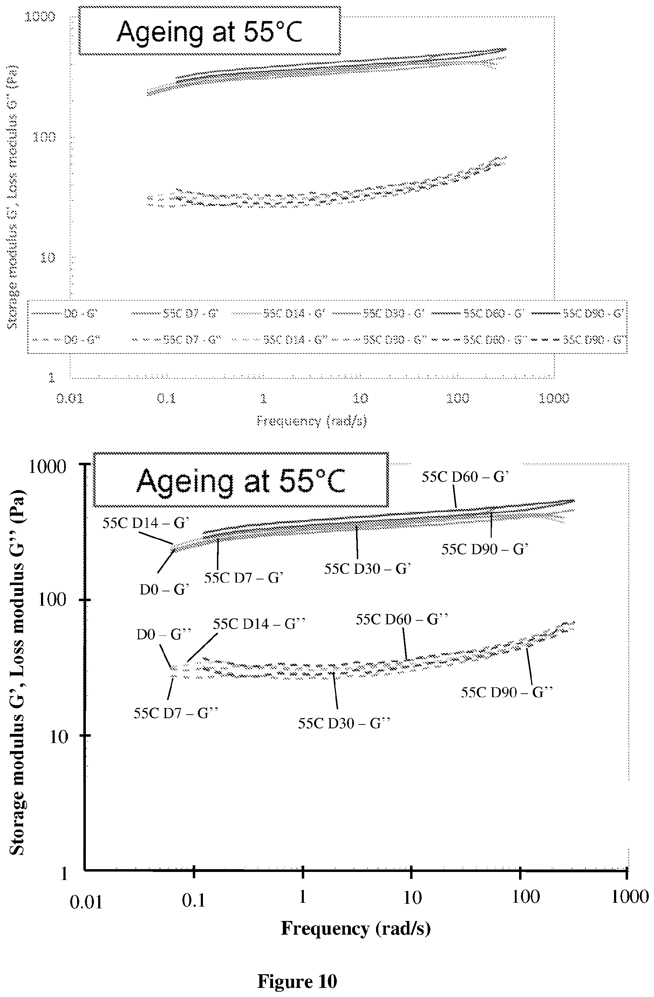

Test conditions were set up to assess the *in-vitro* stability of both MilliQ^{™} purified water and PBS gel preparations. 20 mL glass vials were filled with about 15 mL of DES CNF gel samples, and the vials were sealed with their lids. For each DES CNF sample, 7 glass vials were prepared for each temperature condition (one glass vial was used per time point plus two additional ones for visual checks). Samples were stored either in a fridge at 4 °C, on a bench in the lab at 23 °C or in an oven set at 55 °C for the required length of time. Shelf life studies were performed with: (a) DES CNF 0.8% (w/v) in MilliQ^{™} purified water, and (b) DES CNF 1% (w/v) in PBS; at 4 °C (i.e. refrigerated shelf life), 23 °C (i.e. room temperature shelf life) and 55 °C (i.e. accelerated shelf life), with samples taken at 0 days, and at 7, 14, 30, 60 and 90 days. A study at 50 °C over 7.5 weeks, or at 60 °C over 3.7 weeks approximates 1 year for plastics (General Aging Theory and Simplified Protocol for Accelerated Aging of Medical Devices, Karl J. Hemmerich, July 1, 1998, Testing). The rheology measurements are provided in Figures 8-10. Figure 8 provides ageing results at 4 °C, Figure 9 at 23 °C, and Figure 10 at 55 °C. Figures 8-10 relates to DES CNF 0.8% (w/v) in MilliQ^{™} purified water. Similar results were obtained for DES CNF 1% (w/v) in PBS. In Figures 8-10, D0 is day 0, D7 is day 7, D14 is day 14 and so on.

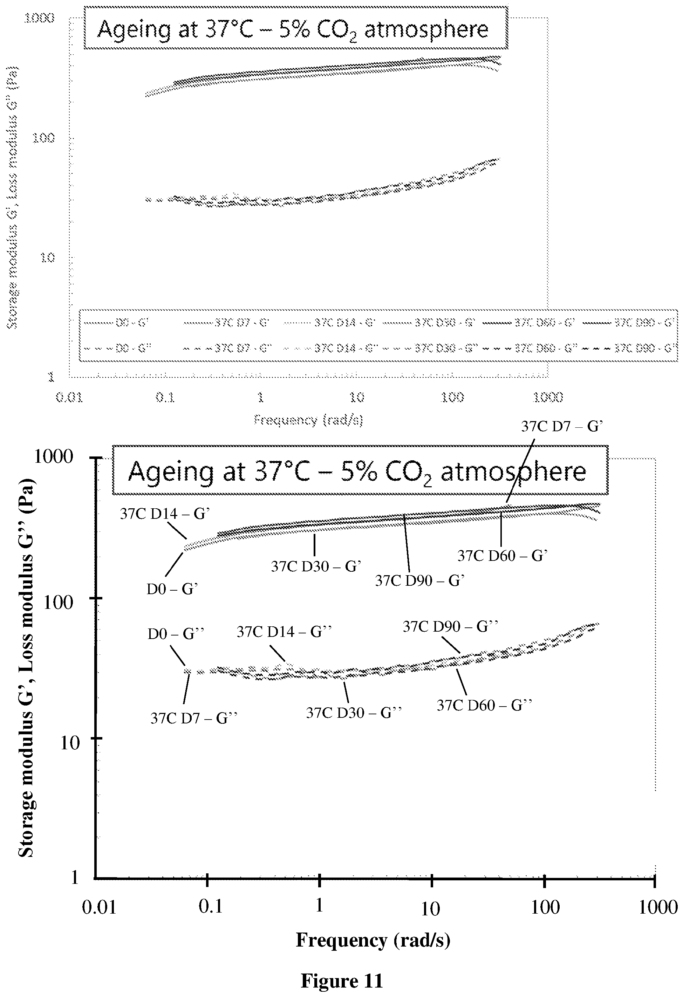

Furthermore, shelf life studies were performed with: (a) DES CNF 0.8% (w/v) in MilliQ^{™} purified water, and (b) DES CNF 1% (w/v) in PBS; at 37 °C (i.e. biological stability) in a 5% CO₂ atmosphere, with samples taken at 0 days, and at 7, 14, 30, 60 and 90 days. In this experiment 20 mL glass vials were filled with about 15 mL DES CNF gel samples, and each lid was loosely closed (i.e. a quarter turn). For each DES CNF sample, 7 glass vials were prepared for each temperature condition (one glass vial was used per time point plus two additional ones for visual checks). Samples were then stored in an incubator at 37 °C under 5 % CO₂ atmosphere for the required length of time. The water dish at the bottom of the incubator was checked every week and toped up with water if necessary. Figure 11 provides ageing results for DES CNF 0.8% (w/v) in MilliQ^{™} purified water. Similar results were obtained for DES CNF 1% (w/v) in PBS.

In the course of these experiments, it was ensured that the samples did not dry out (i.e. samples were topped up with fluid as needed).

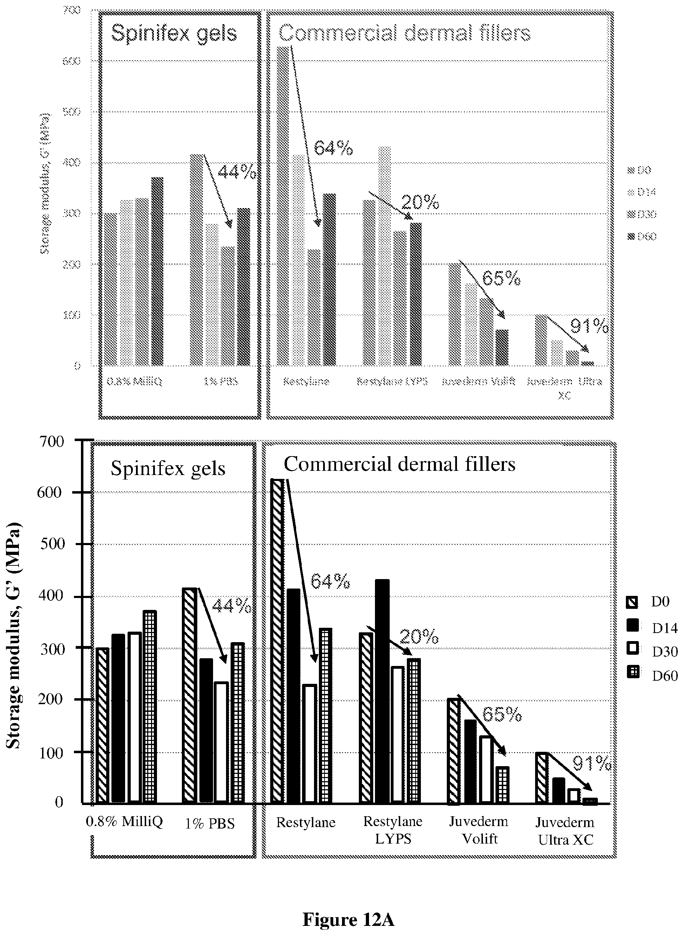

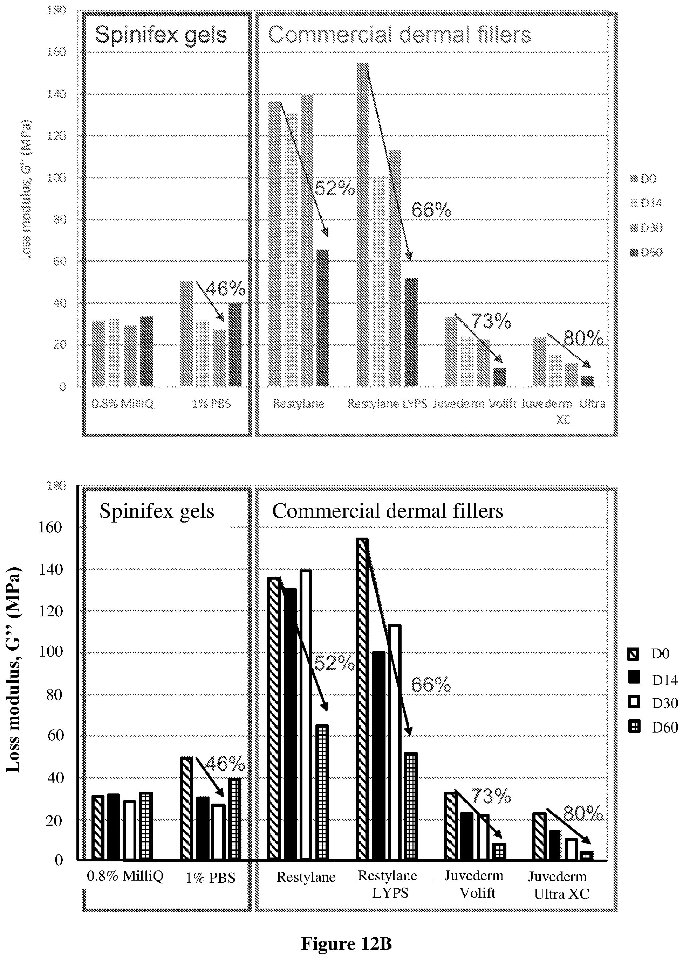

The results were compared with those for commercial dermal fillers, including Restylane^{™}, Restylane^{™} LYPS, Juvéderm^{™} Volift, and Juvéderm^{™} Ultra XC. The results are provided in Figures 12A and 12 B (ageing at 55 °C, G' or G" at 0.1 Hz).

The injectability of the DES CNF samples were also assessed for (a) DES CNF 0.8% (w/v) in MilliQ^{™} purified water, and (b) DES CNF 1% (w/v) in PBS. The Glide Force (N) was assessed for the samples at 0, 7, 14, 30, 60 and 90 days where samples were stored at 4 °C, 23 °C 37 °C under 5% CO₂, and 55 °C. For DES CNF 0.8% (w/v) the glide force at day 0 was about 4.7 N, and over the 90 days this increased to a glide force of between about 5.7 N to 6.7 N. For DES CNF 1% (w/v) in PBS the glide force at day 0 was about 1.6 N, and over the 90 days this increased to a glide force of between about 2.5 N to 3.2 N.

### Example 7: Reversibility Study

Cellulase from *T. reesei* (Sigma; ≥ 700 unit/g) was dissolved in PBS at various concentrations (0.1%, 0.5%, 1% and 2% wt) and was added to DES CNF 1% (w/v) incubated at 37°C. Visual observations were carried out over the incubation period.

The cellulose activity was evaluated by assessing the presence of free carbonyl group (C=O) (reducing sugars) using the DNS (dinitrosalicylic acid) assay at different incubation times (Miller (1959) Use of dinitrosalicylic acid reagent for determination of reducing sugar. Analytical Chemistry, 31, 3 p. 426-428). Rheology was performed on enzymatically degraded DES gels at different incubation times. The results are provided in Table 4 below. As either time or the concentration of cellulase increases, then the rate at which the cellulose breaks down increases.

**Table 4: Glucose content and glucose conversion rate after incubation at 37 °C**

| | Glucose content (µmol/mL) | Glucose conversion rate |
|---|---|---|
| Gel after 1 hour | 0.6 | 0.02% |
| Gel in PBS after 1 hour | 0.3 | 0.01% |
| Gel + 1% cellulase in PBS after 1 hour | 1.1 | 0.04% |
| Gel + 2% cellulase in PBS after 1 hour | 1.6 | 0.06% |
| Gel + 1% cellulase in PBS after 4 hours | 3.1 | 0.12% |
| Gel + 2% cellulase in PBS after 4 hours | 3.8 | 0.13% |

Similarly the storage modulus (G') and the loss modulus (G") for the samples decreases upon exposure to cellulase and is affected by cellulase concentration. For example G' decreased by almost 10 fold after addition of 1 mL of 2% cellulase at 37 °C for 1 hour.

### Example 8: Animal Study

C57BI/6 mice were injected subcutaneously with DES CNF gel (1.0% DES CNF gel in saline), 0.1 ml per injection, 4 different dorsal sites per mouse (left and right flanks, left and right shoulders, with a control (saline) administered on left shoulder and flank, and a sample administered on a right shoulder and flank). Mice received 1.0% DES CNF gel in saline as the sample, or a commercially available hyaluronic acid (HA) (Restylane^{™}) as the sample. The study also included no injection control mice. Mice were monitored daily for signs of irritation or swelling. At weekly intervals, mice were euthanized at days 7, 14, 21, 28 and 56 after gel implantation (n=2 or 3 per time-point). Implanted gel and surrounding tissue were removed for histological analysis to determine the cellular response to the gel. Prior to injection, cellulose was UV irradiated for 50 minutes to sterilise the composition.

After the mice were sacrificed, tissue samples were fixed in 4% paraformaldehyde (PFA) and stained using hematoxylin and eosin (H&E). Where necessary, tissues were placed in 70% ethanol for storage until processing. Histological sections of 6 µm were taken for each sample.

It was observed that the 'softer' cellulose gel (or DES CNF gel) results in a more elongated bolus after 7 days, rather than the HA control (which is more rounded).

After 14 days the HA bolus appeared to lose its 'rounded' shape and its ability to withstand histological processing had decreased (presumably as HA had broken down over time). However, after 14 days the cellulose treatments were mostly intact and were still able to withstand histological processing.

After 56 days, it is estimated that the volume of the bolus for the cellulose samples were around 50% of the original volume at day 0. The bolus in the HA samples were also breaking down.

Whole blood from each mouse was taken at the time of dissection to determine if there was an increase in circulating leukocytes. No differences in the white blood cell count was seen between treatment groups at days 7 and 14. There was no indication of a systemic inflammatory response, and any inflammation would most likely be localised to the injection site.

## Claims

1. A method of cosmetic treatment, the method comprising use of an injectable composition formed from cellulosic fibers and a deep eutectic solvent, wherein the injectable composition is obtainable by a preparation process comprising:
(i) contacting cellulosic fibers with a deep eutectic solvent to provide deep eutectic solvent treated cellulosic fibers;
(ii) washing the deep eutectic solvent treated cellulosic fibers; and
(iii) homogenizing or mechanically refining the washed deep eutectic solvent treated cellulosic fibers to thereby provide the injectable composition.

2. The method of cosmetic treatment of claim **1,** wherein in the preparation process, step (i) is preceded by:
the step of delignifying cellulosic fibers; and
optionally the step of bleaching the delignified cellulosic fibers.

3. The method of cosmetic treatment of claim 1 or claim 2, wherein in the preparation process, the step of contacting the cellulosic nanofibers with a deep eutectic solvent is performed at a temperature of from 120 °C to 180 °C, at a molar ratio of cellulosic nanofibers to deep eutectic solvent of from 1:2 to 1:30.

4. The method of cosmetic treatment of any one of claims 1 to 3, wherein the deep eutectic solvent is formed from a Lewis acid and a Lewis base; wherein the Lewis acid comprises an ammonium, phosphonium or sulfonium cation, an amine, an amide, a carboxylic acid, or a polyol; and wherein the Lewis base comprises a nitrogen atom, or an amide group, a urea group, a carbamate group, or an ammonium group.

5. The method of cosmetic treatment of claim 4, wherein the deep eutectic solvent is sulfamic acid urea.

6. The method of cosmetic treatment of any one of claims 1 to 5, wherein the cellulosic fibers are derived from a plant of the subtribe *Triodiinae.*

7. A cosmetically acceptable injectable composition prepared by the preparation process as defined in any one of claims 1 to 6.

8. Cosmetic use of an injectable composition formed from cellulosic fibers and a deep eutectic solvent as a dermal filler, wherein the composition (i) is obtainable by the preparation process steps as defined in any one of claims 1 to 6; and/or (ii) comprises deep eutectic solvent treated cellulosic fibers; and/or (iii) comprises cellulosic nanofibers complexed with deep eutectic solvent.

9. An injectable composition formed from cellulosic fibers and a deep eutectic solvent for use in plasma rich platelet therapy, wherein the injectable composition further comprises platelets, and wherein the composition (i) is obtainable by the preparation process steps as defined in any one of claims 1 to 6; and/or (ii) comprises deep eutectic solvent treated cellulosic fibers; and/or (iii) comprises cellulosic nanofibers complexed with deep eutectic solvent.

10. A cosmetic method of increasing volume in, or under the skin of, a subject, comprising injecting an injectable composition formed from cellulosic fibers and a deep eutectic solvent in, or under, the skin of the subject, wherein the composition (i) is obtainable by the process steps as defined in any one of claims 1 to 6; and/or (ii) comprises deep eutectic solvent treated cellulosic fibers; and/or (iii) comprises cellulosic nanofibers complexed with deep eutectic solvent.

11. The cosmetic method of claim 10, wherein the method is to increase the volume of the lip, breast or cheek of the subject.

## Patentansprüche

1. Verfahren zur kosmetischen Behandlung, wobei das Verfahren eine Verwendung einer injizierbaren Zusammensetzung, die aus cellulosischen Fasern und einem stark eutektischen Lösungsmittel gebildet ist, umfasst, wobei die injizierbare Zusammensetzung durch einen Zubereitungsprozess erlangbar ist, der Folgendes umfasst:
(i) Inkontaktbringen von cellulosischen Fasern mit einem stark eutektischen Lösungsmittel, um mit einem stark eutektischen Lösungsmittel behandelte cellulosische Fasern bereitzustellen;
(ii) Waschen der mit einem stark eutektischen Lösungsmittel behandelten cellulosischen Fasern; und
(iii) Homogenisieren oder mechanisches Raffinieren der gewaschenen, mit einem stark eutektischen Lösungsmittel behandelten cellulosischen Fasern, um dadurch die injizierbare Zusammensetzung bereitzustellen.

2. Verfahren zur kosmetischen Behandlung nach Anspruch 1, wobei in dem Zubereitungsprozess dem Schritt (i) Folgendes vorausgeht:
der Schritt des Delignifizierens von cellulosischen Fasern; und
optional der Schritt des Bleichens der delignifizierten cellulosischen Fasern.

3. Verfahren zur kosmetischen Behandlung nach Anspruch 1 oder Anspruch 2, wobei in dem Zubereitungsprozess der Schritt des Inkontaktbringens der cellulosischen Nanofasern mit einem stark eutektischen Lösungsmittel bei einer Temperatur von 120 °C bis 180 °C mit einem Molverhältnis von cellulosischen Nanofasern zu einem stark eutektischen Lösungsmittel von 1:2 bis 1:30 durchgeführt wird.

4. Verfahren zur kosmetischen Behandlung nach einem der Ansprüche 1 bis 3, wobei das stark eutektische Lösungsmittel aus einer Lewis-Säure und einer Lewis-Base gebildet ist; wobei die Lewis-Säure ein Ammonium-, Phosphonium- oder Sulfoniumkation, ein Amin, ein Amid, eine Carbonsäure oder ein Polyol umfasst; und wobei die Lewis-Base ein Stickstoffatom oder eine Amidgruppe, eine Harnstoffgruppe, eine Carbamatgruppe oder eine Ammoniumgruppe umfasst.

5. Verfahren zur kosmetischen Behandlung nach Anspruch 4, wobei das stark eutektische Lösungsmittel Amidosulfonsäure-Harnstoff ist.

6. Verfahren zur kosmetischen Behandlung nach einem der Ansprüche 1 bis 5, wobei die cellulosischen Fasern aus einer Pflanze der Untertribus Triodiinae abgeleitet sind.

7. Kosmetisch verträgliche injizierbare Zusammensetzung, die durch den Zubereitungsprozess wie in einem der Ansprüche 1 bis 6 definiert hergestellt wird.

8. Kosmetische Verwendung einer injizierbaren Zusammensetzung, die aus cellulosischen Fasern und einem stark eutektischen Lösungsmittel gebildet ist, als Dermalfüller, wobei die Zusammensetzung (i) durch die Zubereitungsprozessschritte, wie in einem der Ansprüche 1 bis 6 definiert, erlangbar ist; und/oder (ii) mit einem stark eutektischen Lösungsmittel behandelte cellulosische Fasern umfasst; und/oder (iii) cellulosische Nanofasern umfasst, die mit einem stark eutektischen Lösungsmittel komplexiert sind.

9. Injizierbare Zusammensetzung, die aus cellulosischen Fasern und einem stark eutektischen Lösungsmittel gebildet ist, zur Verwendung in einer Therapie mit plättchenreichem Plasma, wobei die injizierbare Zusammensetzung ferner Plättchen umfasst, und wobei die Zusammensetzung (i) durch die Zubereitungsprozessschritte, wie in einem der Ansprüche 1 bis 6 definiert, erlangbar ist; und/oder (ii) mit einem stark eutektischen Lösungsmittel behandelte cellulosische Fasern umfasst; und/oder (iii) cellulosische Nanofasern umfasst, die mit einem stark eutektischen Lösungsmittel komplexiert sind.

10. Kosmetisches Verfahren zum Erhöhen von Volumen in oder unter der Haut eines Subjekts, umfassend Injizieren einer injizierbaren Zusammensetzung, die aus cellulosischen Fasern und einem stark eutektischen Lösungsmittel gebildet ist, in oder unter die Haut des Subjekts, wobei die Zusammensetzung (i) durch die Prozessschritte, wie in einem der Ansprüche 1 bis 6 definiert, erlangbar ist; und/oder (ii) mit einem stark eutektischen Lösungsmittel behandelte cellulosische Fasern umfasst; und/oder (iii) cellulosische Nanofasern umfasst, die mit einem stark eutektischen Lösungsmittel komplexiert sind.

11. Kosmetisches Verfahren nach Anspruch 10, wobei das Verfahren dazu dient, das Volumen der Lippe, der Brust oder der Wange des Subjekts zu erhöhen.

## Revendications

1. Procédé de traitement cosmétique, le procédé comprenant l'utilisation d'une composition injectable formée à partir de fibres cellulosiques et d'un solvant eutectique profond, dans lequel la composition injectable peut être obtenue par un processus de préparation comprenant :
(i) la mise en contact de fibres cellulosiques avec un solvant eutectique profond pour la fourniture de fibres cellulosiques traitées par solvant eutectique profond ;
(ii) le lavage des fibres cellulosiques traitées par solvant eutectique profond ; et
(iii) l'homogénéisation ou le raffinage mécanique des fibres cellulosiques traitées par solvant eutectique profond lavées pour la fourniture ainsi de la composition injectable.

2. Procédé de traitement cosmétique selon la revendication 1, dans lequel, dans le processus de préparation, l'étape (i) est précédée par :
l'étape de délignification de fibres cellulosiques ; et
éventuellement l'étape de blanchiment des fibres cellulosiques délignifiées.

3. Procédé de traitement cosmétique selon la revendication 1 ou 2, dans lequel, dans le processus de préparation, l'étape de mise en contact des nanofibres cellulosiques avec un solvant eutectique profond est réalisée à une température allant de 120 °C à 180 °C, à un rapport molaire des nanofibres cellulosiques au solvant eutectique profond allant de 1:2 à 1:30.

4. Procédé de traitement cosmétique selon l'une quelconque des revendications 1 à 3, dans lequel le solvant eutectique profond est formé à partir d'un acide de Lewis et d'une base de Lewis ; dans lequel l'acide de Lewis comprend un cation ammonium, phosphonium ou sulfonium, une amine, un amide, un acide carboxylique ou un polyol ; et dans lequel la base de Lewis comprend un atome d'azote, ou un groupe amide, un groupe urée, un groupe carbamate ou un groupe ammonium.

5. Procédé de traitement cosmétique selon la revendication 4, dans lequel le solvant eutectique profond est l'acide sulfamique-urée.

6. Procédé de traitement cosmétique selon l'une quelconque des revendications 1 à 5, dans lequel les fibres cellulosiques sont dérivées d'une plante de la sous-tribu des *Triodiinae*.

7. Composition injectable cosmétiquement acceptable préparée par le processus de préparation tel que défini dans l'une quelconque des revendications 1 à 6.

8. Utilisation cosmétique d'une composition injectable formée à partir de fibres cellulosiques et d'un solvant eutectique profond en tant que produit de comblement dermique, dans laquelle la composition (i) peut être obtenue par les étapes de processus de préparation telles que définies dans l'une quelconque des revendications 1 à 6 ; et/ou (ii) comprend des fibres cellulosiques traitées par solvant eutectique profond ; et/ou (iii) comprend des nanofibres cellulosiques complexées avec un solvant eutectique profond.

9. Composition injectable formée à partir de fibres cellulosiques et d'un solvant eutectique profond pour l'utilisation dans une thérapie par plasma riche en plaquettes, dans laquelle la composition injectable comprend en outre des plaquettes, et dans laquelle la composition (i) peut être obtenue par les étapes de processus de préparation telles que définies dans l'une quelconque des revendications 1 à 6 ; et/ou (ii) comprend des fibres cellulosiques traitées par solvant eutectique profond ; et/ou (iii) comprend des nanofibres cellulosiques complexées avec un solvant eutectique profond.

10. Procédé cosmétique d'augmentation de volume dans la peau, ou sous la peau, d'un sujet, comprenant l'injection d'une composition injectable formée à partir de fibres cellulosiques et d'un solvant eutectique profond dans la peau, ou sous la peau, du sujet, dans lequel la composition (i) peut être obtenue par les étapes de processus telles que définies dans l'une quelconque des revendications 1 à 6 ; et/ou (ii) comprend des fibres cellulosiques traitées par solvant eutectique profond ; et/ou (iii) comprend des nanofibres cellulosiques complexées avec un solvant eutectique profond.

11. Procédé cosmétique selon la revendication 10, dans lequel le procédé est pour l'augmentation du volume de la lèvre, du sein ou de la joue du sujet.
